# EUROPEAN PATENT SPECIFICATION

(11) **EP 0 988 373 B1**
(45) Date of publication and mention of the grant of the patent: **21.02.2007**
(21) Application number: 98928753.7
(22) Date of filing: 04.06.1998
(51) Int. Cl.: C12N 9/14, C12N 15/67

(54) **SELECTION MARKER**
SELEKTIONSMARKER
MARQUEUR DE SELECTION

(30) Priority: 04.06.1997 SE 9702120
(43) Date of publication of application: 29.03.2000
(73) Proprietor: Avaris AB, 171 77 Stockholm (SE)
(72) Inventor: BELUSA, Roger, S-181 33 Lidingö (SE)
(74) Representative: Larfeldt, Helene
(86) International application number: PCT/SE1998/001062
(87) International publication number: WO 1998/055603

(56) References cited:
- EP-A- 0 068 763
- STN INTERNATIONAL, FILE CAPLUS, CAPLUS Accession No. 1988:144404, HERRERA VICTORIA et al., "Three Differentially Expressed Sodium-Potassium ATPase .alpha. Subunit Isoforms: Structural and Functional Implications"; & J. CELL. BIOL., (1997), 105(4), p. 1855-65.
- STN INTERNATIONAL, FILE CAPLUS, CAPLUS Accession No. 1996:412147, BURNS EDINA L. et al., "Random Mutagenesis of the Sheep Na,K-ATPase .alpha.1 Subunit Generating the Ouabain-Resistant Mutant L793P"; & J. BIOL. CHEM., (1996), 271(27), p. 15879-15883.
- CHEMICAL ABSTRACTS, Volume 119, No. 21, 22 November 1993, (Columbus, Ohio, USA), BURNS EDINA L. et al., "Random Mutagenesis of the Sheep Sodium-Potasium-ATPase alpha-1 Subunit Generates a Novel T797N Mutation That Results in a Ouabain-Resistant Enzyme", page 437, Abstract No. 220422e; & J. BIOL. CHEM., 1993, 268(34), p. 25632-25635.
- CHEMICAL ABSTRACTS, Volume 108, No. 25, 20 June 1988, (Columbus, Ohio, USA), DUDANI ANIL KUMAR et al., "Absence of Gene Amplification in Human Cell Mutants Resistant to Cardiac Glycosides", page 27, Abstract No. 215939x; & MOL. CELL. BIOCHEM., 1987, 78(1), p. 73-79.

### SEQUENCE LISTING

(1) GENERAL INFORMATION:
   (i) APPLICANT:
      (A) NAME: Karolinska Innovation
      (B) STREET: Tomtebodav 11F
      (C) CITY: Stockholm
      (E) COUNTRY: Sweden
      (F) POSTAL CODE (ZIP): 171 77
      (G) TELEPHONE: 08-7286510
      (H) TELEFAX: 08-303423
   (ii) TITLE OF INVENTION: NEW SELECTION MARKER (mutated NKA alphal subunit)
   (iii) NUMBER OF SEQUENCES: 8
   (iv) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.30 (EPO)
(2) INFORMATION FOR SEQ ID NO: 1:
   (i) SEQUENCE CHARACTERISTICS:
      (A) LENGTH: 1212 amino acids
      (B) TYPE: amino acid
      (C) STRANDEDNESS:
      (D) TOPOLOGY: linear
   (ii) MOLECULE TYPE: protein
   (iii) HYPOTHETICAL: NO
   (iv) ANTI-SENSE: NO
   (v) FRAGMENT TYPE: N-terminal.
   (vi) ORIGINAL SOURCE:
      (A) ORGANISM: Rattus rattus
      (C) INDIVIDUAL ISOLATE: rat
   (xi) SEQUENCE DESCRIPTION: SEQ ID NO: 1:
   Gly Ala Ser Ala Gly Gly Gly Gly His Val Ala Ala Ala Ala Ala Ala
   1 5 10 15
   Ala Ala Ala Ala Ala Ala Ala Ala Ala Ala Ser Val Arg Gly Ala Gly
   20 25 30
   Arg Pro Pro Ser Phe Leu Arg Arg Gln Pro Xaa Phe Pro Pro Leu Gly
   35 40 45
   Ser Pro Gly Ser Thr Leu Pro Ala Gly Ser Cys Ser Leu Leu Phe Leu
   50 55 60
   Val Ser Ser His Arg Thr Arg Arg Gly Ala Arg Ser Ala Ala Thr Met
   65 70 75 80
   Gly Lys Gly Val Gly Arg Asp Lys Tyr Glu Pro Ala Ala Val Ser Glu
   85 90 95
   His Gly Asp Lys Lys Ser Lys Lys Ala Lys Lys Glu Arg Asp Met Asp
   100 105 110
   Glu Leu Lys Lys Glu Val Ser Met Asp Asp His Lys Leu Ser Leu Asp
   115 120 125
   Glu Leu His Arg Lys Tyr Gly Thr Asp Leu Ser Arg Gly Leu Thr Pro
   130 135 140
   Ala Arg Ala Ala Glu Ile Leu Ala Arg Asp Gly Pro Asn Ala Leu Thr
   145 150 155 160
   Pro Pro Pro Thr Thr Pro Glu Trp Val Lys Phe Cys Arg Gln Leu Phe
   165 170 175
   Gly Gly Phe Ser Met Leu Leu Trp Ile Gly Ala Ile Leu Cys Phe Leu
   180 185 190
   Ala Tyr Gly Ile Arg Ser Ala Thr Glu Glu Glu Pro Pro Asn Asp Asp
   195 200 205
   Leu Tyr Leu Gly Val Val Leu Ser Ala Val Val Ile Ile Thr Gly Cys
   210 215 220
   Phe Ser Tyr Tyr Gln Glu Ala Lys Ser Ser Lys Ile Met Glu Ser Phe
   225 230 235 240
   Lys Asn Met Val Pro Gln Gln Ala Leu Val Ile Arg Asn Gly Glu Lys
   245 250 255
   Met Ser Ile Asn Ala Glu Asp Val Val Val Gly Asp Leu Val Glu Val
   260 265 270
   Lys Gly Gly Asp Arg Ile Pro Ala Asp Leu Arg Ile Ile Ser Ala Asn
   275 280 285
   Gly Cys Lys Val Asp Asn Ser Ser Leu Thr Gly Glu Ser Glu Pro Gln
   290 295 300
   Thr Arg Ser Pro Asp Phe Thr Asn Glu Asn Pro Leu Glu Thr Arg Asn
   305 310 315 320
   Ile Ala Phe Phe Ser Thr Asn Cys Val Glu Gly Thr Ala Arg Gly Ile
   325 330 335
   Val Val Tyr Thr Gly Asp Arg Thr Val Met Gly Arg Ile Ala Thr Leu
   340 345 350
   Ala Ser Gly Leu Glu Gly Gly Gln Thr Pro Ile Ala Glu Glu Ile Glu
   355 360 365
   His Phe Ile His Leu Ile Thr Gly Val Ala Val Phe Leu Gly Val Ser
   370 375 380
   Phe Phe Ile Leu Ser Leu Ile Leu Glu Tyr Thr Trp Leu Glu Ala Val
   385 390 395 400
   Ile Phe Leu Ile Gly Ile Ile Val Ala Asn Val Pro Glu Gly Leu Leu
   405 410 415
   Ala Thr Val Thr Val Cys Leu Thr Leu Thr Ala Lys Arg Met Ala Arg
   420 425 430
   Lys Asn Cys Leu Val Lys Asn Leu Glu Ala Val Glu Thr Leu Gly Ser
   435 440 445
   Thr Ser Thr Ile Cys Ser Asp Lys Thr Gly Thr Leu Thr Gln Asn Arg
   450 455 460
   Met Thr Val Ala His Met Trp Phe Asp Asn Gln Ile His Glu Ala Asp
   465 470 475 480
   Thr Thr Glu Asn Gln Ser Gly Val Ser Phe Asp Lys Thr Ser Ala Thr
   485 490 495
   Trp Phe Ala Leu Ser Arg Ile Ala Gly Leu Cys Asn Arg Ala Val Phe
   500 505 510
   Gln Ala Asn Gln Glu Asn Leu Pro Ile Leu Lys Arg Ala Val Ala Gly
   515 520 525
   Asp Ala Ser Glu Ser Ala Leu Leu Lys Cys Ile Glu Val Cys Cys Gly
   530 535 540
   Ser Val Met Glu Met Arg Glu Lys Tyr Thr Lys Ile Val Glu Ile Pro
   545 550 555 560
   Phe Asn Ser Thr Asn Lys Tyr Gln Leu Ser Ile His Lys Asn Pro Asn
   565 570 575
   Ala Ser Glu Pro Lys His Leu Leu Val Met Lys Gly Ala Pro Glu Arg
   580 585 590
   Ile Leu Asp Arg Cys Ser Ser Ile Leu Leu His Gly Lys Glu Gln Pro
   595 600 605
   Leu Asp Glu Glu Leu Lys Asp Ala Phe Gln Asn Ala Tyr Leu Glu Leu
   610 615 620
   Gly Gly Leu Gly Glu Arg Val Leu Gly Phe Cys His Leu Leu Leu Pro
   625 630 635 640
   Asp Glu Gln Phe Pro Glu Gly Phe Gln Phe Asp Thr Asp Glu Val Asn
   645 650 655
   Phe Pro Val Asp Asn Leu Cys Phe Val Gly Leu Ile Ser Met Ile Asp
   660 665 670
   Pro Pro Arg Ala Ala Val Pro Asp Ala Val Gly Lys Cys Arg Ser Ala
   675 680 685
   Gly Ile Lys Val Ile Met Val Thr Gly Asp His Pro Ile Thr Ala Lys
   690 695 700
   Ala Ile Ala Lys Gly Val Gly Ile Ile Ser Glu Gly Asn Glu Thr Val
   705 710 715 720
   Glu Asp Ile Ala Ala Arg Leu Asn Ile Pro Val Asn Gln Val Asn Pro
   725 730 735
   Arg Asp Ala Lys Ala Cys Val Val His Gly Ser Asp Leu Lys Asp Met
   740 745 750
   Thr Ser Glu Glu Leu Asp Asp Ile Leu Arg Tyr His Thr Glu Ile Val
   755 760 765
   Phe Ala Arg Thr Ser Pro Gln Gln Lys Leu Ile Ile Val Glu Gly Cys
   770 775 780
   Gln Arg Gln Gly Ala Ile Val Ala Val Thr Gly Asp Gly Val Asn Asp
   785 790 795 800
   Ser Pro Ala Leu Lys Lys Ala Asp Ile Gly Val Ala Met Gly Ile Val
   805 810 815
   Gly Ser Asp Val Ser Lys Gln Ala Ala Asp Met Ile Leu Leu Asp Asp
   820 825 830
   Asn Phe Ala Ser Ile Val Thr Gly Val Glu Glu Gly Arg Leu Ile Phe
   835 840 845
   Asp Asn Leu Lys Lys Ser Ile Ala Tyr Thr Leu Thr Ser Asn Ile Pro
   850 855 860
   Glu Ile Thr Pro Phe Leu Ile Phe Ile Ile Ala Asn Ile Pro Cys Pro
   865 870 875 880
   Leu Gly Thr Val Thr Ile Leu Cys Ile Asp Leu Gly Thr Asp Met Val
   885 890 895
   Pro Ala Ile Ser Leu Ala Tyr Glu Gln Ala Glu Ser Asp Ile Met Lys
   900 905 910
   Arg Gln Pro Arg Asn Pro Lys Thr Asp Lys Leu Val Asn Glu Arg Leu
   915 920 925
   Ile Ser Met Ala Tyr Gly Gln Ile Gly Met Ile Gln Ala Leu Gly Gly
   930 935 940
   Phe Phe Thr Tyr Phe Val Ile Leu Ala Glu Asn Gly Phe Leu Pro Phe
   945 950 955 960
   His Leu Leu Gly Ile Arg Glu Thr Trp Asp Asp Arg Trp Ile Asn Asp
   965 970 975
   Val Glu Asp Ser Tyr Gly Gln Gln Trp Thr Tyr Glu Gln Arg Lys Ile
   980 985 990
   Val Glu Phe Thr Cys His Thr Ala Phe Phe Val Ser Ile Val Val Val
   995 1000 1005
   Gln Trp Ala Asp Leu Val Ile Cys Lys Thr Arg Arg Asn Ser Val Phe
   1010 1015 1020
   Gln Gln Gly Met Lys Asn Lys Ile Leu Ile Phe Gly Leu Phe Glu Glu
   1025 1030 1035 1040
   Thr Ala Leu Ala Ala Phe Leu Ser Tyr Cys Pro Gly Met Gly Ala Ala
   1045 1050 1055
   Leu Arg Met Tyr Pro Leu Lys Pro Thr Trp Trp Phe Cys Ala Phe Pro
   1060 1065 1070
   Tyr Ser Leu Leu Ile Phe Val Tyr Asp Glu Val Arg Lys Leu Ile Ile
   1075 1080 1085
   Arg Arg Arg Pro Gly Gly Trp Val Glu Lys Glu Thr Tyr Tyr Xaa Pro
   1090 1095 1100
   Thr Ala Leu His Ala Val Glu His Cys Ala Thr His Cys Thr Tyr Pro
   1105 1110 1115 1120
   Tyr Pro Pro Phe Val Tyr Phe Lys Ser Trp Ser Ser Glu Leu Tyr Pro
   1125 1130 1135
   Gly Arg Lys Ala Pro Lys His Val Gly Ile Gln Thr Ser Trp Asn Glu
   1140 1145 1150
   Ala Cys Ser Cys Asn Gly Gly Arg Gly Glu Gly Cys Pro Lys Asn Thr
   1155 1160 1165
   Val Asp Gly Asp Asp Ser Gly Glu Gly Leu Tyr Val Pro Phe Cys Phe
   1170 1175 1180
   Cys Lys Lys Gly Lys Pro Gly Lys Thr Glu Arg Leu Arg Phe Ile Ser
   1185 1190 1195 1200
   Gly Phe Leu Gln Ile Lys Met Ala Ile Ile Thr Glu
   1205 1210
(2) INFORMATION FOR SEQ ID NO: 2:
   (i) SEQUENCE CHARACTERISTICS:
      (A) LENGTH: 1212 amino acids
      (B) TYPE: amino acid
      (C) STRANDEDNESS:
      (D) TOPOLOGY: linear
   (ii) MOLECULE TYPE: protein
   (iii) HYPOTHETICAL: NO
   (iv) ANTI-SENSE: NO
   (v) FRAGMENT TYPE: N-terminal
   (vi) ORIGINAL SOURCE:
      (A) ORGANISM: NKA alpha 1 subunit
      (C) INDIVIDUAL ISOLATE: rat
      (G) CELL TYPE: rat
   (xi) SEQUENCE DESCRIPTION: SEQ ID NO: 2:
   Gly Ala Ser Ala Gly Gly Gly Gly His Val Ala Ala Ala Ala Ala Ala
   1 5 10 15
   Ala Ala Ala Ala Ala Ala Ala Ala Ala Ala Ser Val Arg Gly Ala Gly
   20 25 30
   Arg Pro Pro Ser Phe Leu Arg Arg Gln Pro Xaa Phe Pro Pro Leu Gly
   35 40 45
   Ser Pro Gly Ser Thr Leu Pro Ala Gly Ser Cys Ser Leu Leu Phe Leu
   50 55 60
   Val Ser Ser His Arg Thr Arg Arg Gly Ala Arg Ser Ala Ala Thr Met
   65 70 75 80
   Gly Lys Gly Val Gly Arg Asp Lys Tyr Glu Pro Ala Ala Val Ser Glu
   85 90 95
   His Gly Asp Lys Lys Ser Lys Lys Ala Lys Lys Glu Arg Asp Met Asp
   100 105 110
   Glu Leu Lys Lys Glu Val Ser Met Asp Asp His Lys Leu Ser Leu Asp
   115 120 125
   Glu Leu His Arg Lys Tyr Gly Thr Asp Leu Ser Arg Gly Leu Thr Pro
   130 135 140
   Ala Arg Ala Ala Glu Ile Leu Ala Arg Asp Gly Pro Asn Ala Leu Thr
   145 150 155 160
   Pro Pro Pro Thr Thr Pro Glu Trp Val Lys Phe Cys Arg Gln Leu Phe
   165 170 175
   Gly Gly Phe Ser Met Leu Leu Trp Ile Gly Ala Ile Leu Cys Phe Leu
   180 185 190
   Ala Tyr Gly Ile Arg Ser Ala Thr Glu Glu Glu Pro Pro Asn Asp Asp
   195 200 205
   Leu Tyr Leu Gly Val Val Leu Ser Ala Val Val Ile Ile Thr Gly Cys
   210 215 220
   Phe Ser Tyr Tyr Gln Glu Ala Lys Ser Ser Lys Ile Met Glu Ser Phe
   225 230 235 240
   Lys Asn Met Val Pro Gln Gln Ala Leu Val Ile Arg Asn Gly Glu Lys
   245 250 255
   Met Ser Ile Asn Ala Glu Asp Val Val Val Gly Asp Leu Val Glu Val
   260 265 270
   Lys Gly Gly Asp Arg Ile Pro Ala Asp Leu Arg Ile Ile Ser Ala Asn
   275 280 285
   Gly Cys Lys Val Asp Asn Ser Ser Leu Thr Gly Glu Ser Glu Pro Gln
   290 295 300
   Thr Arg Ser Pro Asp Phe Thr Asn Glu Asn Pro Leu Glu Thr Arg Asn
   305 310 315 320
   Ile Ala Phe Phe Ser Thr Asn Cys Val Glu Gly Thr Ala Arg Gly Ile
   325 330 335
   Val Val Tyr Thr Gly Asp Arg Thr Val Met Gly Arg Ile Ala Thr Leu
   340 345 350
   Ala Ser Gly Leu Glu Gly Gly Gln Thr Pro Ile Ala Glu Glu Ile Glu
   355 360 365
   His Phe Ile His Leu Ile Thr Gly Val Ala Val Phe Leu Gly Val Ser
   370 375 380
   Phe Phe Ile Leu Ser Leu Ile Leu Glu Tyr Thr Trp Leu Glu Ala Val
   385 390 395 400
   Ile Phe Leu Ile Gly Ile Ile Val Ala Asn Val Pro Glu Gly Leu Leu
   405 410 415
   Ala Thr Val Thr Val Cys Leu Thr Leu Thr Ala Lys Arg Met Ala Arg
   420 425 430
   Lys Asn Cys Leu Val Lys Asn Leu Glu Ala Val Glu Thr Leu Gly Ser
   435 440 445
   Thr Ser Thr Ile Cys Ser Asp Lys Thr Gly Thr Leu Thr Gln Asn Arg
   450 455 460
   Met Thr Val Ala His Met Trp Phe Asp Asn Gln Ile His Glu Ala Asp
   465 470 475 480
   Thr Thr Glu Asn Gln Ser Gly Val Ser Phe Asp Lys Thr Ser Ala Thr
   485 490 495
   Trp Phe Ala Leu Ser Arg Ile Ala Gly Leu Cys Asn Arg Ala Val Phe
   500 505 510
   Gln Ala Asn Gln Glu Asn Leu Pro Ile Leu Lys Arg Ala Val Ala Gly
   515 520 525
   Asp Ala Ser Glu Ser Ala Leu Leu Lys Cys Ile Glu Val Cys Cys Gly
   530 535 540
   Ser Val Met Glu Met Arg Glu Lys Tyr Thr Lys Ile Val Glu Ile Pro
   545 550 555 560
   Phe Asn Ser Thr Asn Lys Tyr Gln Leu Ser Ile His Lys Asn Pro Asn
   565 570 575
   Ala Ser Glu Pro Lys His Leu Leu Val Met Lys Gly Ala Pro Glu Arg
   580 585 590
   Ile Leu Asp Arg Cys Ser Ser Ile Leu Leu His Gly Lys Glu Gln Pro
   595 600 605
   Leu Asp Glu Glu Leu Lys Asp Ala Phe Gln Asn Ala Tyr Leu Glu Leu
   610 615 620
   Gly Gly Leu Gly Glu Arg Val Leu Gly Phe Cys His Leu Leu Leu Pro
   625 630 635 640
   Asp Glu Gln Phe Pro Glu Gly Phe Gln Phe Asp Thr Asp Glu Val Asn
   645 650 655
   Phe Pro Val Asp Asn Leu Cys Phe Val Gly Leu Ile Ser Met Ile Asp
   660 665 670
   Pro Pro Arg Ala Ala Val Pro Asp Ala Val Gly Lys Cys Arg Ser Ala
   675 680 685
   Gly Ile Lys Val Ile Met Val Thr Gly Asp His Pro Ile Thr Ala Lys
   690 695 700
   Ala Ile Ala Lys Gly Val Gly Ile Ile Ser Glu Gly Asn Glu Thr Val
   705 710 715 720
   Glu Asp Ile Ala Ala Arg Leu Asn Ile Pro Val Asn Gln Val Asn Pro
   725 730 735
   Arg Asp Ala Lys Ala Cys Val Val His Gly Ser Asp Leu Lys Asp Met
   740 745 750
   Thr Ser Glu Glu Leu Asp Asp Ile Leu Arg Tyr His Thr Glu Ile Val
   755 760 765
   Phe Ala Arg Thr Ser Pro Gln Gln Lys Leu Ile Ile Val Glu Gly Cys
   770 775 780
   Gln Arg Gln Gly Ala Ile Val Ala Val Thr Gly Asp Gly Val Asn Asp
   785 790 795 800
   Ser Pro Ala Leu Lys Lys Ala Asp Ile Gly Val Ala Met Gly Ile Val
   805 810 815
   Gly Ser Asp Val Ser Lys Gln Ala Ala Asp Met Ile Leu Leu Asp Asp
   820 825 830
   Asn Phe Ala Ser Ile Val Thr Gly Val Glu Glu Gly Arg Leu Ile Phe
   835 840 845
   Asp Asn Leu Lys Lys Ser Ile Ala Tyr Thr Leu Thr Ser Asn Ile Pro
   850 855 860
   Glu Ile Thr Pro Phe Leu Ile Phe Ile Ile Ala Asn Ile Pro Leu Pro
   865 870 875 880
   Cys Gly Thr Val Thr Ile Leu Cys Ile Asp Leu Gly Thr Asp Met Val
   885 890 895
   Pro Ala Ile Ser Leu Ala Tyr Glu Gln Ala Glu Ser Asp Ile Met Lys
   900 905 910
   Arg Gln Pro Arg Asn Pro Lys Thr Asp Lys Leu Val Asn Glu Arg Leu
   915 920 925
   Ile Ser Met Ala Tyr Gly Gln Ile Gly Met Ile Gln Ala Leu Gly Gly
   930 935 940
   Phe Phe Thr Tyr Phe Val Ile Leu Ala Glu Asn Gly Phe Leu Pro Phe
   945 950 955 960
   His Leu Leu Gly Ile Arg Glu Thr Trp Asp Asp Arg Trp Ile Asn Asp
   965 970 975
   Val Glu Asp Ser Tyr Gly Gln Gln Trp Thr Tyr Glu Gln Arg Lys Ile
   980 985 990
   Val Glu Phe Thr Cys His Thr Ala Phe Phe Val Ser Ile Val Val Val
   995 1000 1005
   Gln Trp Ala Asp Leu Val Ile Cys Lys Thr Arg Arg Asn Ser Val Phe
   1010 1015 1020
   Gln Gln Gly Met Lys Asn Lys Ile Leu Ile Phe Gly Leu Phe Glu Glu
   1025 1030 1035 1040
   Thr Ala Leu Ala Ala Phe Leu Ser Tyr Cys Pro Gly Met Gly Ala Ala
   1045 1050 1055
   Leu Arg Met Tyr Pro Leu Lys Pro Thr Trp Trp Phe Cys Ala Phe Pro
   1060 1065 1070
   Tyr Ser Leu Leu Ile Phe Val Tyr Asp Glu Val Arg Lys Leu Ile Ile
   1075 1080 1085
   Arg Arg Arg Pro Gly Gly Trp Val Glu Lys Glu Thr Tyr Tyr Xaa Pro
   1090 1095 1100
   Thr Ala Leu His Ala Val Glu His Cys Ala Thr His Cys Thr Tyr Pro
   1105 1110 1115 1120
   Tyr Pro Pro Phe Val Tyr Phe Lys Ser Trp Ser Ser Glu Leu Tyr Pro
   1125 1130 1135
   Gly Arg Lys Ala Pro Lys His Val Gly Ile Gln Thr Ser Trp Asn Glu
   1140 1145 1150
   Ala Cys Ser Cys Asn Gly Gly Arg Gly Glu Gly Cys Pro Lys Asn Thr
   1155 1160 1165
   Val Asp Gly Asp Asp Ser Gly Glu Gly Leu Tyr Val Pro Phe Cys Phe
   1170 1175 1180
   Cys Lys Lys Gly Lys Pro Gly Lys Thr Glu Arg Leu Arg Phe Ile Ser
   1185 1190 1195 1200
   Gly Phe Leu Gln Ile Lys Met Ala Ile Ile Thr Glu
   1205 1210
(2) INFORMATION FOR SEQ ID NO: 3:
   (i) SEQUENCE CHARACTERISTICS:
      (A) LENGTH: 1212 amino acids
      (B) TYPE: amino acid
      (C) STRANDEDNESS:
      (D) TOPOLOGY: linear
   (ii) MOLECULE TYPE: protein
   (iii) HYPOTHETICAL: NO
   (iv) ANTI-SENSE: NO
   (v) FRAGMENT TYPE: N-terminal
   (vi) ORIGINAL SOURCE:
      (A) ORGANISM: NKA alpha 1 subunit
      (C) INDIVIDUAL ISOLATE: rat
      (G) CELL TYPE: rat
   (xi) SEQUENCE DESCRIPTION: SEQ ID NO: 3:
   Gly Ala Ser Ala Gly Gly Gly Gly His Val Ala Ala Ala Ala Ala Ala
   1 5 10 15
   Ala Ala Ala Ala Ala Ala Ala Ala Ala Ala Ser Val Arg Gly Ala Gly
   20 25 30
   Arg Pro Pro Ser Phe Leu Arg Arg Gln Pro Xaa Phe Pro Pro Leu Gly
   35 40 45
   Ser Pro Gly Ser Thr Leu Pro Ala Gly Ser Cys Ser Leu Leu Phe Leu
   50 55 60
   Val Ser Ser His Arg Thr Arg Arg Gly Ala Arg Ser Ala Ala Thr Met
   65 70 75 80
   Gly Lys Gly Val Gly Arg Asp Lys Tyr Glu Pro Ala Ala Val Ser Glu
   85 90 95
   His Gly Asp Lys Lys Ser Lys Lys Ala Lys Lys Glu Arg Asp Met Asp
   100 105 110
   Glu Leu Lys Lys Glu Val Ser Met Asp Asp His Lys Leu Ser Leu Asp
   115 120 125
   Glu Leu His Arg Lys Tyr Gly Thr Asp Leu Ser Arg Gly Leu Thr Pro
   130 135 140
   Ala Arg Ala Ala Glu Ile Leu Ala Arg Asp Gly Pro Asn Ala Leu Thr
   145 150 155 160
   Pro Pro Pro Thr Thr Pro Glu Trp Val Lys Phe Cys Arg Gln Leu Phe
   165 170 175
   Gly Gly Phe Ser Met Leu Leu Trp Ile Gly Ala Ile Leu Cys Phe Leu
   180 185 190
   Ala Tyr Gly Ile Arg Ser Ala Thr Glu Glu Glu Pro Pro Asn Asp Asp
   195 200 205
   Leu Tyr Leu Gly Val Val Leu Ser Ala Val Val Ile Ile Thr Gly Cys
   210 215 220
   Phe Ser Tyr Tyr Gln Glu Ala Lys Ser Ser Lys Ile Met Glu Ser Phe
   225 230 235 240
   Lys Asn Met Val Pro Gln Gln Ala Leu Val Ile Arg Asn Gly Glu Lys
   245 250 255
   Met Ser Ile Asn Ala Glu Asp Val Val Val Gly Asp Leu Val Glu Val
   260 265 270
   Lys Gly Gly Asp Arg Ile Pro Ala Asp Leu Arg Ile Ile Ser Ala Asn
   275 280 285
   Gly Cys Lys Val Asp Asn Ser Ser Leu Thr Gly Glu Ser Glu Pro Gln
   290 295 300
   Thr Arg Ser Pro Asp Phe Thr Asn Glu Asn Pro Leu Glu Thr Arg Asn
   305 310 315 320
   Ile Ala Phe Phe Ser Thr Asn Cys Val Glu Gly Thr Ala Arg Gly Ile
   325 330 335
   Val Val Tyr Thr Gly Asp Arg Thr Val Met Gly Arg Ile Ala Thr Leu
   340 345 350
   Ala Ser Gly Leu Glu Gly Gly Gln Thr Pro Ile Ala Glu Glu Ile Glu
   355 360 365
   His Phe Ile His Leu Ile Thr Gly Val Ala Val Phe Leu Gly Val Ser
   370 375 380
   Phe Phe Ile Leu Ser Leu Ile Leu Glu Tyr Thr Trp Leu Glu Ala Val
   385 390 395 400
   Ile Phe Leu Ile Gly Ile Ile Val Ala Asn Val Pro Glu Gly Leu Leu
   405 410 415
   Ala Thr Val Thr Val Cys Leu Thr Leu Thr Ala Lys Arg Met Ala Arg
   420 425 430
   Lys Asn Cys Leu Val Lys Asn Leu Glu Ala Val Glu Thr Leu Gly Ser
   435 440 445
   Thr Ser Thr Ile Cys Ser Asp Lys Thr Gly Thr Leu Thr Gln Asn Arg
   450 455 460
   Met Thr Val Ala His Met Trp Phe Asp Asn Gln Ile His Glu Ala Asp
   465 470 475 480
   Thr Thr Glu Asn Gln Ser Gly Val Ser Phe Asp Lys Thr Ser Ala Thr
   485 490 495
   Trp Phe Ala Leu Ser Arg Ile Ala Gly Leu Cys Asn Arg Ala Val Phe
   500 505 510
   Gln Ala Asn Gln Glu Asn Leu Pro Ile Leu Lys Arg Ala Val Ala Gly
   515 520 525
   Asp Ala Ser Glu Ser Ala Leu Leu Lys Cys Ile Glu Val Cys Cys Gly
   530 535 540
   Ser Val Met Glu Met Arg Glu Lys Tyr Thr Lys Ile Val Glu Ile Pro
   545 550 555 560
   Phe Asn Ser Thr Asn Lys Tyr Gln Leu Ser Ile His Lys Asn Pro Asn
   565 570 575
   Ala Ser Glu Pro Lys His Leu Leu Val Met Lys Gly Ala Pro Glu Arg
   580 585 590
   Ile Leu Asp Arg Cys Ser Ser Ile Leu Leu His Gly Lys Glu Gln Pro
   595 600 605
   Leu Asp Glu Glu Leu Lys Asp Ala Phe Gln Asn Ala Tyr Leu Glu Leu
   610 615 620
   Gly Gly Leu Gly Glu Arg Val Leu Gly Phe Cys His Leu Leu Leu Pro
   625 630 635 640
   Asp Glu Gln Phe Pro Glu Gly Phe Gln Phe Asp Thr Asp Glu Val Asn
   645 650 655
   Phe Pro Val Asp Asn Leu Cys Phe Val Gly Leu Ile Ser Met Ile Asp
   660 665 670
   Pro Pro Arg Ala Ala Val Pro Asp Ala Val Gly Lys Cys Arg Ser Ala
   675 680 685
   Gly Ile Lys Val Ile Met Val Thr Gly Asp His Pro Ile Thr Ala Lys
   690 695 700
   Ala Ile Ala Lys Gly Val Gly Ile Ile Ser Glu Gly Asn Glu Thr Val
   705 710 715 720
   Glu Asp Ile Ala Ala Arg Leu Asn Ile Pro Val Asn Gln Val Asn Pro
   725 730 735
   Arg Asp Ala Lys Ala Cys Val Val His Gly Ser Asp Leu Lys Asp Met
   740 745 750
   Thr Ser Glu Glu Leu Asp Asp Ile Leu Arg Tyr His Thr Glu Ile Val
   755 760 765
   Phe Ala Arg Thr Ser Pro Gln Gln Lys Leu Ile Ile Val Glu Gly Cys
   770 775 780
   Gln Arg Gln Gly Ala Ile Val Ala Val Thr Gly Asp Gly Val Asn Asp
   785 790 795 800
   Ser Pro Ala Leu Lys Lys Ala Asp Ile Gly Val Ala Met Gly Ile Val
   805 810 815
   Gly Ser Asp Val Ser Lys Gln Ala Ala Asp Met Ile Leu Leu Asp Asp
   820 825 830
   Asn Phe Ala Ser Ile Val Thr Gly Val Glu Glu Gly Arg Leu Ile Phe
   835 840 845
   Asp Asn Leu Lys Lys Ser Ile Ala Tyr Thr Leu Thr Ser Asn Ile Pro
   850 855 860
   Glu Ile Thr Pro Phe Leu Ile Phe Ile Ile Ala Asn Ile Pro Cys Pro
   865 870 875 880
   Cys Gly Thr Val Thr Ile Leu Cys Ile Asp Leu Gly Thr Asp Met Val
   885 890 895
   Pro Ala Ile Ser Leu Ala Tyr Glu Gln Ala Glu Ser Asp Ile Met Lys
   900 905 910
   Arg Gln Pro Arg Asn Pro Lys Thr Asp Lys Leu Val Asn Glu Arg Leu
   915 920 925
   Ile Ser Met Ala Tyr Gly Gln Ile Gly Met Ile Gln Ala Leu Gly Gly
   930 935 940
   Phe Phe Thr Tyr Phe Val Ile Leu Ala Glu Asn Gly Phe Leu Pro Phe
   945 950 955 960
   His Leu Leu Gly Ile Arg Glu Thr Trp Asp Asp Arg Trp Ile Asn Asp
   965 970 975
   Val Glu Asp Ser Tyr Gly Gln Gln Trp Thr Tyr Glu Gln Arg Lys Ile
   980 985 990
   Val Glu Phe Thr Cys His Thr Ala Phe Phe Val Ser Ile Val Val Val
   995 1000 1005
   Gln Trp Ala Asp Leu Val Ile Cys Lys Thr Arg Arg Asn Ser Val Phe
   1010 1015 1020
   Gln Gln Gly Met Lys Asn Lys Ile Leu Ile Phe Gly Leu Phe Glu Glu
   1025 1030 1035 1040
   Thr Ala Leu Ala Ala Phe Leu Ser Tyr Cys Pro Gly Met Gly Ala Ala
   1045 1050 1055
   Leu Arg Met Tyr Pro Leu Lys Pro Thr Trp Trp Phe Cys Ala Phe Pro
   1060 1065 1070
   Tyr Ser Leu Leu Ile Phe Val Tyr Asp Glu Val Arg Lys Leu Ile Ile
   1075 1080 1085
   Arg Arg Arg Pro Gly Gly Trp Val Glu Lys Glu Thr Tyr Tyr Xaa Pro
   1090 1095 1100
   Thr Ala Leu His Ala Val Glu His Cys Ala Thr His Cys Thr Tyr Pro
   1105 1110 1115 1120
   Tyr Pro Pro Phe Val Tyr Phe Lys Ser Trp Ser Ser Glu Leu Tyr Pro
   1125 1130 1135
   Gly Arg Lys Ala Pro Lys His Val Gly Ile Gln Thr Ser Trp Asn Glu
   1140 1145 1150
   Ala Cys Ser Cys Asn Gly Gly Arg Gly Glu Gly Cys Pro Lys Asn Thr
   1155 1160 1165
   Val Asp Gly Asp Asp Ser Gly Glu Gly Leu Tyr Val Pro Phe Cys Phe
   1170 1175 1180
   Cys Lys Lys Gly Lys Pro Gly Lys Thr Glu Arg Leu Arg Phe Ile Ser
   1185 1190 1195 1200
   Gly Phe Leu Gln Ile Lys Met Ala Ile Ile Thr Glu
   1205 1210
(2) INFORMATION FOR SEQ ID NO: 4:
   (i) SEQUENCE CHARACTERISTICS:
      (A) LENGTH: 1212 amino acids
      (B) TYPE: amino acid
      (C) STRANDEDNESS:
      (D) TOPOLOGY: linear
   (ii) MOLECULE TYPE: protein
   (iii) HYPOTHETICAL: NO
   (iv) ANTI-SENSE: NO
   (v) FRAGMENT TYPE: N-terminal
   (vi) ORIGINAL SOURCE:
      (A) ORGANISM: NKA alpha 1 subunit
      (C) INDIVIDUAL ISOLATE: rat
      (G) CELL TYPE: rat
   (xi) SEQUENCE DESCRIPTION: SEQ ID NO: 4:
   Gly Ala Ser Ala Gly Gly Gly Gly His Val Ala Ala Ala Ala Ala Ala
   1               5                   10                  15
   Ala Ala Ala Ala Ala Ala Ala Ala Ala Ala Ser Val Arg Gly Ala Gly
               20                  25                  30
   Arg Pro Pro Ser Phe Leu Arg Arg Gln Pro Xaa Phe Pro Pro Leu Gly
           35                  40                  45
   Ser Pro Gly Ser Thr Leu Pro Ala Gly Ser Cys Ser Leu Leu Phe Leu
       50                  55                  60
   Val Ser Ser His Arg Thr Arg Arg Gly Ala Arg Ser Ala Ala Thr Met
   65                  70                  75                  80
   Gly Lys Gly Val Gly Arg Asp Lys Tyr Glu Pro Ala Ala Val Ser Glu
                   85                  90                  95
   His Gly Asp Lys Lys Ser Lys Lys Ala Lys Lys Glu Arg Asp Met Asp
               100                 105                 110
   Glu Leu Lys Lys Glu Val Ser Met Asp Asp His Lys Leu Ser Leu Asp
           115                 120                 125
   Glu Leu His Arg Lys Tyr Gly Thr Asp Leu Ser Arg Gly Leu Thr Pro
       130                 135                 140
   Ala Arg Ala Ala Glu Ile Leu Ala Arg Asp Gly Pro Asn Ala Leu Thr
   145                 150                 155                 160
   Pro Pro Pro Thr Thr Pro Glu Trp Val Lys Phe Cys Arg Gln Leu Phe
                   165                 170                 175
   Gly Gly Phe Ser Met Leu Leu Trp Ile Gly Ala Ile Leu Cys Phe Leu
   180 185 190
   Ala Tyr Gly Ile Arg Ser Ala Thr Glu Glu Glu Pro Pro Asn Asp Asp
   195 200 205
   Leu Tyr Leu Gly Val Val Leu Ser Ala Val Val Ile Ile Thr Gly Cys
   210 215 220
   Phe Ser Tyr Tyr Gln Glu Ala Lys Ser Ser Lys Ile Met Glu Ser Phe
   225 230 235 240
   Lys Asn Met Val Pro Gln Gln Ala Leu Val Ile Arg Asn Gly Glu Lys
   245 250 255
   Met Ser Ile Asn Ala Glu Asp Val Val Val Gly Asp Leu Val Glu Val
   260 265 270
   Lys Gly Gly Asp Arg Ile Pro Ala Asp Leu Arg Ile Ile Ser Ala Asn
   275 280 285
   Gly Cys Lys Val Asp Asn Ser Ser Leu Thr Gly Glu Ser Glu Pro Gln
   290 295 300
   Thr Arg Ser Pro Asp Phe Thr Asn Glu Asn Pro Leu Glu Thr Arg Asn
   305 310 315 320
   Ile Ala Phe Phe Ser Thr Asn Cys Val Glu Gly Thr Ala Arg Gly Ile
   325 330 335
   Val Val Tyr Thr Gly Asp Arg Thr Val Met Gly Arg Ile Ala Thr Leu
   340 345 350
   Ala Ser Gly Leu Glu Gly Gly Gln Thr Pro Ile Ala Glu Glu Ile Glu
   355 360 365
   His Phe Ile His Leu Ile Thr Gly Val Ala Val Phe Leu Gly Val Ser
   370 375 380
   Phe Phe Ile Leu Ser Leu Ile Leu Glu Tyr Thr Trp Leu Glu Ala Val
   385 390 395 400
   Ile Phe Leu Ile Gly Ile Ile Val Ala Asn Val Pro Glu Gly Leu Leu
   405 410 415
   Ala Thr Val Thr Val Cys Leu Thr Leu Thr Ala Lys Arg Met Ala Arg
   420 425 430
   Lys Asn Cys Leu Val Lys Asn Leu Glu Ala Val Glu Thr Leu Gly Ser
   435 440 445
   Thr Ser Thr Ile Cys Ser Asp Lys Thr Gly Thr Leu Thr Gln Asn Arg
   450 455 460
   Met Thr Val Ala His Met Trp Phe Asp Asn Gln Ile His Glu Ala Asp
   465 470 475 480
   Thr Thr Glu Asn Gln Ser Gly Val Ser Phe Asp Lys Thr Ser Ala Thr
   485 490 495
   Trp Phe Ala Leu Ser Arg Ile Ala Gly Leu Cys Asn Arg Ala Val Phe
   500 505 510
   Gln Ala Asn Gln Glu Asn Leu Pro Ile Leu Lys Arg Ala Val Ala Gly
   515 520 525
   Asp Ala Ser Glu Ser Ala Leu Leu Lys Cys Ile Glu Val Cys Cys Gly
   530 535 540
   Ser Val Met Glu Met Arg Glu Lys Tyr Thr Lys Ile Val Glu Ile Pro
   545 550 555 560
   Phe Asn Ser Thr Asn Lys Tyr Gln Leu Ser Ile His Lys Asn Pro Asn
   565 570 575
   Ala Ser Glu Pro Lys His Leu Leu Val Met Lys Gly Ala Pro Glu Arg
   580 585 590
   Ile Leu Asp Arg Cys Ser Ser Ile Leu Leu His Gly Lys Glu Gln Pro
   595 600 605
   Leu Asp Glu Glu Leu Lys Asp Ala Phe Gln Asn Ala Tyr Leu Glu Leu
   610 615 620
   Gly Gly Leu Gly Glu Arg Val Leu Gly Phe Cys His Leu Leu Leu Pro
   625 630 635 640
   Asp Glu Gln Phe Pro Glu Gly Phe Gln Phe Asp Thr Asp Glu Val Asn
   645 650 655
   Phe Pro Val Asp Asn Leu Cys Phe Val Gly Leu Ile Ser Met Ile Asp
   660 665 670
   Pro Pro Arg Ala Ala Val Pro Asp Ala Val Gly Lys Cys Arg Ser Ala
   675 680 685
   Gly Ile Lys Val Ile Met Val Thr Gly Asp His Pro Ile Thr Ala Lys
   690 695 700
   Ala Ile Ala Lys Gly Val Gly Ile Ile Ser Glu Gly Asn Glu Thr Val
   705 710 715 720
   Glu Asp Ile Ala Ala Arg Leu Asn Ile Pro Val Asn Gln Val Asn Pro
   725 730 735
   Arg Asp Ala Lys Ala Cys Val Val His Gly Ser Asp Leu Lys Asp Met
   740 745 750
   Thr Ser Glu Glu Leu Asp Asp Ile Leu Arg Tyr His Thr Glu Ile Val
   755 760 765
   Phe Ala Arg Thr Ser Pro Gln Gln Lys Leu Ile Ile Val Glu Gly Cys
   770 775 780
   Gln Arg Gln Gly Ala Ile Val Ala Val Thr Gly Asp Gly Val Asn Asp
   785 790 795 800
   Ser Pro Ala Leu Lys Lys Ala Asp Ile Gly Val Ala Met Gly Ile Val
   805 810 815
   Gly Ser Asp Val Ser Lys Gln Ala Ala Asp Met Ile Leu Leu Asp Asp
   820 825 830
   Asn Phe Ala Ser Ile Val Thr Gly Val Glu Glu Gly Arg Leu Ile Phe
   835 840 845
   Asp Asn Leu Lys Lys Ser Ile Ala Tyr Thr Leu Thr Ser Asn Ile Pro
   850 855 860
   Glu Ile Thr Pro Phe Leu Ile Phe Ile Ile Ala Asn Ile Pro Leu Pro
   865 870 875 880
   Leu Gly Thr Val Thr Ile Leu Cys Ile Asp Leu Gly Thr Asp Met Val
   885 890 895
   Pro Ala Ile Ser Leu Ala Tyr Glu Gln Ala Glu Ser Asp Ile Met Lys
   900 905 910
   Arg Gln Pro Arg Asn Pro Lys Thr Asp Lys Leu Val Asn Glu Arg Leu
   915 920 925
   Ile Ser Met Ala Tyr Gly Gln Ile Gly Met Ile Gln Ala Leu Gly Gly
   930 935 940
   Phe Phe Thr Tyr Phe Val Ile Leu Ala Glu Asn Gly Phe Leu Pro Phe
   945 950 955 960
   His Leu Leu Gly Ile Arg Glu Thr Trp Asp Asp Arg Trp Ile Asn Asp
   965 970 975
   Val Glu Asp Ser Tyr Gly Gln Gln Trp Thr Tyr Glu Gln Arg Lys Ile
   980 985 990
   Val Glu Phe Thr Cys His Thr Ala Phe Phe Val Ser Ile Val Val Val
   995 1000 1005
   Gln Trp Ala Asp Leu Val Ile Cys Lys Thr Arg Arg Asn Ser Val Phe
   1010 1015 1020
   Gln Gln Gly Met Lys Asn Lys Ile Leu Ile Phe Gly Leu Phe Glu Glu
   1025 1030 1035 1040
   Thr Ala Leu Ala Ala Phe Leu Ser Tyr Cys Pro Gly Met Gly Ala Ala
   1045 1050 1055
   Leu Arg Met Tyr Pro Leu Lys Pro Thr Trp Trp Phe Cys Ala Phe Pro
   1060 1065 1070
   Tyr Ser Leu Leu Ile Phe Val Tyr Asp Glu Val Arg Lys Leu Ile Ile
   1075 1080 1085
   Arg Arg Arg Pro Gly Gly Trp Val Glu Lys Glu Thr Tyr Tyr Xaa Pro
   1090 1095 1100
   Thr Ala Leu His Ala Val Glu His Cys Ala Thr His Cys Thr Tyr Pro
   1105 1110 1115 1120
   Tyr Pro Pro Phe Val Tyr Phe Lys Ser Trp Ser Ser Glu Leu Tyr Pro
   1125 1130 1135
   Gly Arg Lys Ala Pro Lys His Val Gly Ile Gln Thr Ser Trp Asn Glu
   1140 1145 1150
   Ala Cys Ser Cys Asn Gly Gly Arg Gly Glu Gly Cys Pro Lys Asn Thr
   1155 1160 1165
   Val Asp Gly Asp Asp Ser Gly Glu Gly Leu Tyr Val Pro Phe Cys Phe
   1170 1175 1180
   Cys Lys Lys Gly Lys Pro Gly Lys Thr Glu Arg Leu Arg Phe Ile Ser
   1185 1190 1195 1200
   Gly Phe Leu Gln Ile Lys Met Ala Ile Ile Thr Glu
   1205 1210
(2) INFORMATION FOR SEQ ID NO: 5:
   (i) SEQUENCE CHARACTERISTICS:
      (A) LENGTH: 3636 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Rattus rattus
      (C) INDIVIDUAL ISOLATE: RAT
   (xi) SEQUENCE DESCRIPTION: SEQ ID NO: 5:
   GGAGCCTCGG CGGGAGGAGG CGGACACGTG GCAGCGGCGG CGGCAGCGGC AGCAGCAGCG    60
   GCGGCAGCAG CGGCGGCCTC GGTCCGGGGC GCCGGCCGTC CTCCCTCTTT CCTCCGGCGG 120
   CAGCCCTAGT TCCCGCCTCT CGGCTCCCCC GGCTCCACTC TCCCAGCCGG GAGCTGCTCT 180
   CTCCTCTTTC TAGTCTCCAG CCACAGGACC CGGCGCGGGG CCCGCAGCGC CGCCACCATG 240
   GGGAAGGGGG TTGGACGAGA CAAGTATGAG CCCGCAGCTG TATCAGAACA TGGGGACAAG 300
   AAGAGCAAGA AGGCGAAGAA GGAAAGGGAC ATGGACGAAC TCAAGAAGGA AGTGTCTATG 360
   GACGACCATA AACTCAGCCT GGATGAACTC CATCGTAAAT ACGGAACAGA TTTGAGCCGA 420
   GGCCTAACAC CCGCAAGGGC CGCTGAGATC CTGGCTCGGG ATGGCCCCAA CGCCCTCACG 480
   CCCCCTCCCA CTACTCCCGA GTGGGTCAAA TTCTGTCGGC AGCTGTTCGG TGGCTTCTCC 540
   ATGTTACTGT GGATTGGAGC CATTCTTTGT TTCTTGGCTT ATGGCATCCG AAGTGCTACA 600
   GAAGAGGAAC CACCAAATGA TGATCTGTAC CTCGGGGTCG TGCTGTCTGC TGTCGTCATC 660
   ATAACTGGCT GTTTCTCCTA TTATCAAGAA GCAAAAAGCT CCAAGATCAT GGAATCCTTC 720
   AAGAACATGG TCCCTCAGCA AGCCCTCGTG ATTCGAAATG GAGAGAAGAT GAGCATCAAC 780
   GCAGAGGATG TCGTCGTTGG TGATCTGGTG GAGGTGAAGG GCGGAGACCG AATCCCTGCT 840
   GATCTCAGAA TCATATCTGC AAATGGCTGC AAGGTGGATA ACTCCTCACT CACTGGTGAA 900
   TCAGAACCCC AGACTCGGTC CCCGGATTTC ACAAACGAGA ACCCCTTGGA GACAAGGAAC 960
   ATTGCCTTCT TCTCAACCAA CTGTGTTGAA GGAACTGCAC GTGGCATCGT TGTGTACACT 1020
   GGGGATCGCA CCGTGATGGG CAGGATCGCC ACCCTTGCTT CTGGGCTGGA AGGCGGCCAG 1080
   ACCCCCATTG CTGAAGAAAT CGAGCACTTC ATCCACCTCA TCACGGGTGT GGCCGTGTTC 1140
   CTGGGGGTGT CTTTCTTCAT TCTCTCTCTG ATCCTTGAGT ACACCTGGCT CGAGGCTGTC 1200
   ATCTTCCTCA TTGGTATCAT CGTAGCCAAC GTGCCGGAAG GTTTGCTGGC CACCGTCACG 1260
   GTATGTCTGA CGCTCACTGC CAAGCGCATG GCGAGGAAGA ACTGCCTGGT GAAGAACCTG 1320
   GAAGCTGTGG AGACCTTGGG GTCCACATCC ACCATCTGCT CCGACAAGAC TGGAACTCTG 1380
   ACTCAGAACC GGATGACAGT GGCTCACATG TGGTTTGACA ATCAAATCCA TGAAGCTGAC 1440
   ACCACAGAGA ATCAGAGTGG GGTCTCCTTT GACAAGACGT CAGCCACCTG GTTCGCTCTG 1500
   TCCAGAATTG CTGGTCTCTG TAACAGGGCA GTGTTTCAGG CTAACCAAGA AAACCTGCCT 1560
   ATCCTTAAGC GTGCAGTAGC GGGAGATGCT TCCGAGTCGG CGCTCTTAAA GTGCATCGAG 1620
   GTCTGCTGTG GCTCCGTGAT GGAGATGAGG GAGAAGTACA CCAAGATAGT GGAGATTCCT 1680
   TTCAACTCCA CCAACAAGTA CCAGCTCTCC ATTCACAAGA ACCCAAACGC ATCGGAGCCT 1740
   AAGCACCTGC TAGTGATGAA GGGCGCCCCA GAAAGGATCC TGGACCGATG CAGTTCTATC 1800
   CTCCTCCACG GCAAGGAGCA GCCCCTGGAC GAAGAGCTGA AGGACGCCTT TCAGAATGCC 1860
   TACCTAGAGC TGGGGGGCCT TGGAGAGCGT GTGCTAGGTT TCTGCCACCT CCTTCTGCCT 1920
   GACGAACAGT TTCCCGAAGG CTTCCAGTTT GACACTGATG AAGTCAATTT CCCCGTGGAT 1980
   AACCTCTGCT TCGTGGGTCT TATCTCCATG ATTGACCCTC CTCGAGCTGC TGTCCCCGAT 2040
   GCTGTGGGCA AATGCCGCAG CGCTGGGATT AAGGTCATCA TGGTCACAGG AGACCATCCA 2100
   ATCACAGCCA AAGCCATTGC TAAGGGGGTG GGCATTATCT CAGAAGGTAA CGAGACCGTG 2160
   GAAGACATTG CTGCCCGCCT CAACATTCCA GTGAACCAGG TGAACCCCAG AGATGCCAAG 2220
   GCCTGTGTAG TACATGGCAG TGACTTGAAG GACATGACCT CTGAGGAGCT GGATGACATT 2280
   TTGCGGTACC ACACGGAGAT TGTCTTTGCT AGGACCTCTC CTCAACAGAA GCTCATCATT 2340
   GTGGAGGGCT GCCAGCGGCA GGGTGCCATC GTGGCTGTCA CAGGGGATGG TGTCAATGAC 2400
   TCTCCAGCTT TGAAAAAGGC AGATATTGGG GTTGCCATGG GGATTGTTGG CTCGGATGTG 2460
   TCCAAGCAAG CTGCTGACAT GATTCTTCTG GATGACAACT TTGCCTCCAT CGTGACTGGA 2520
   GTAGAAGAAG GTCGTCTGAT ATTTGATAAC TTGAAGAAAT CCATTGCTTA CACCCTAACA 2580
   AGTAACATTC CGGAAATCAC CCCCTTCTTG ATATTTATTA TTGCAAACAT TCCATGTCCC 2640
   CTGGGCACCG TGACCATCCT CTGCATTGAC TTGGGCACTG ACATGGTTCC CGCCATCTCT 2700
   CTGGCCTATG AACAGGCTGA AAGTGACATC ATGAAGAGGC AGCCCAGAAA TCCCAAAACG 2760
   GACAAACTTG TGAACGAGCG TCTGATCAGC ATGGCCTATG GACAGATCGG TATGATCCAG 2820
   GCCCTGGGAG GCTTCTTCAC TTATTTTGTG ATTCTGGCTG AGAACGGTTT CCTGCCCTTT 2880
   CACCTGTTGG GCATCCGAGA GACCTGGGAT GACCGCTGGA TCAATGATGT GGAGGACAGC 2940
   TACGGGCAGC AGTGGACCTA CGAGCAGAGG AAGATTGTGG AGTTCACCTG CCACACGGCC 3000
   TTCTTTGTCA GTATCGTGGT AGTGCAGTGG GCTGACTTGG TCATCTGCAA GACCAGAAGG 3060
   AATTCTGTCT TCCAGCAGGG AATGAAGAAC AAGATCTTAA TATTTGGCCT CTTTGAAGAG 3120
   ACAGCTCTTG CTGCTTTCCT GTCCTACTGC CCTGGGATGG GTGCAGCCCT TAGGATGTAT 3180
   CCCCTCAAAC CTACTTGGTG GTTCTGTGCC TTCCCCTACT CCCTTCTCAT CTTCGTGTAT 3240
   GACGAGGTGC GGAAGCTCAT CATCAGGCGA CGCCCTGGCG GCTGGGTGGA GAAGGAAACC 3300
   TACTACTAGC CCACTGCCCT GCACGCCGTG GAACATTGTG CCACACACTG CACCTACCCC 3360
   TACCCCCCCT TTGTGTACTT CAAGTCTTGG AGCTCGGAAC TCTACCCTGG TAGGAAAGCA 3420
   CCAAAGCATG TGGGGATCCA GACGTCCTGG AATGAAGCAT GTAGCTGTAA TGGGGGGCGG 3480
   GGGGAGGGCT GCCCGAAAAA CACCGTGGAC GGGGACGACA GCGGGGAAGG TTTATATGTG 3540
   CCTTTTTGTT TTTGTAAAAA AGGAAAACCT GGAAAGACTG AAAGATTACG TTTTATATCT 3600
   GGATTTTTAC AAATAAAGAT GGCTATTATA ACGGAA 3636
(2) INFORMATION FOR SEQ ID NO: 6:
   (i) SEQUENCE CHARACTERISTICS:
      (A) LENGTH: 3636 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Rattus rattus
      (C) INDIVIDUAL ISOLATE: RAT
   (xi) SEQUENCE DESCRIPTION: SEQ ID NO: 6:
   GGAGCCTCGG CGGGAGGAGG CGGACACGTG GCAGCGGCGG CGGCAGCGGC AGCAGCAGCG 60
   GCGGCAGCAG CGGCGGCCTC GGTCCGGGGC GCCGGCCGTC CTCCCTCTTT CCTCCGGCGG 120
   CAGCCCTAGT TCCCGCCTCT CGGCTCCCCC GGCTCCACTC TCCCAGCCGG GAGCTGCTCT 180
   CTCCTCTTTC TAGTCTCCAG CCACAGGACC CGGCGCGGGG CCCGCAGCGC CGCCACCATG 240
   GGGAAGGGGG TTGGACGAGA CAAGTATGAG CCCGCAGCTG TATCAGAACA TGGGGACAAG 300
   AAGAGCAAGA AGGCGAAGAA GGAAAGGGAC ATGGACGAAC TCAAGAAGGA AGTGTCTATG 360
   GACGACCATA AACTCAGCCT GGATGAACTC CATCGTAAAT ACGGAACAGA TTTGAGCCGA 420
   GGCCTAACAC CCGCAAGGGC CGCTGAGATC CTGGCTCGGG ATGGCCCCAA CGCCCTCACG 480
   CCCCCTCCCA CTACTCCCGA GTGGGTCAAA TTCTGTCGGC AGCTGTTCGG TGGCTTCTCC 540
   ATGTTACTGT GGATTGGAGC CATTCTTTGT TTCTTGGCTT ATGGCATCCG AAGTGCTACA 600
   GAAGAGGAAC CACCAAATGA TGATCTGTAC CTCGGGGTCG TGCTGTCTGC TGTCGTCATC 660
   ATAACTGGCT GTTTCTCCTA TTATCAAGAA GCAAAAAGCT CCAAGATCAT GGAATCCTTC 720
   AAGAACATGG TCCCTCAGCA AGCCCTCGTG ATTCGAAATG GAGAGAAGAT GAGCATCAAC 780
   GCAGAGGATG TCGTCGTTGG TGATCTGGTG GAGGTGAAGG GCGGAGACCG AATCCCTGCT 840
   GATCTCAGAA TCATATCTGC AAATGGCTGC AAGGTGGATA ACTCCTCACT CACTGGTGAA 900
   TCAGAACCCC AGACTCGGTC CCCGGATTTC ACAAACGAGA ACCCCTTGGA GACAAGGAAC 960
   ATTGCCTTCT TCTCAACCAA CTGTGTTGAA GGAACTGCAC GTGGCATCGT TGTGTACACT 1020
   GGGGATCGCA CCGTGATGGG CAGGATCGCC ACCCTTGCTT CTGGGCTGGA AGGCGGCCAG 1080
   ACCCCCATTG CTGAAGAAAT CGAGCACTTC ATCCACCTCA TCACGGGTGT GGCCGTGTTC 1140
   CTGGGGGTGT CTTTCTTCAT TCTCTCTCTG ATCCTTGAGT ACACCTGGCT CGAGGCTGTC 1200
   ATCTTCCTCA TTGGTATCAT CGTAGCCAAC GTGCCGGAAG GTTTGCTGGC CACCGTCACG 1260
   GTATGTCTGA CGCTCACTGC CAAGCGCATG GCGAGGAAGA ACTGCCTGGT GAAGAACCTG 1320
   GAAGCTGTGG AGACCTTGGG GTCCACATCC ACCATCTGCT CCGACAAGAC TGGAACTCTG 1380
   ACTCAGAACC GGATGACAGT GGCTCACATG TGGTTTGACA ATCAAATCCA TGAAGCTGAC 1440
   ACCACAGAGA ATCAGAGTGG GGTCTCCTTT GACAAGACGT CAGCCACCTG GTTCGCTCTG 1500
   TCCAGAATTG CTGGTCTCTG TAACAGGGCA GTGTTTCAGG CTAACCAAGA AAACCTGCCT 1560
   ATCCTTAAGC GTGCAGTAGC GGGAGATGCT TCCGAGTCGG CGCTCTTAAA GTGCATCGAG 1620
   GTCTGCTGTG GCTCCGTGAT GGAGATGAGG GAGAAGTACA CCAAGATAGT GGAGATTCCT 1680
   TTCAACTCCA CCAACAAGTA CCAGCTCTCC ATTCACAAGA ACCCAAACGC ATCGGAGCCT 1740
   AAGCACCTGC TAGTGATGAA GGGCGCCCCA GAAAGGATCC TGGACCGATG CAGTTCTATC 1800
   CTCCTCCACG GCAAGGAGCA GCCCCTGGAC GAAGAGCTGA AGGACGCCTT TCAGAATGCC 1860
   TACCTAGAGC TGGGGGGCCT TGGAGAGCGT GTGCTAGGTT TCTGCCACCT CCTTCTGCCT 1920
   GACGAACAGT TTCCCGAAGG CTTCCAGTTT GACACTGATG AAGTCAATTT CCCCGTGGAT 1980
   AACCTCTGCT TCGTGGGTCT TATCTCCATG ATTGACCCTC CTCGAGCTGC TGTCCCCGAT 2040
   GCTGTGGGCA AATGCCGCAG CGCTGGGATT AAGGTCATCA TGGTCACAGG AGACCATCCA 2100
   ATCACAGCCA AAGCCATTGC TAAGGGGGTG GGCATTATCT CAGAAGGTAA CGAGACCGTG 2160
   GAAGACATTG CTGCCCGCCT CAACATTCCA GTGAACCAGG TGAACCCCAG AGATGCCAAG 2220
   GCCTGTGTAG TACATGGCAG TGACTTGAAG GACATGACCT CTGAGGAGCT GGATGACATT 2280
   TTGCGGTACC ACACGGAGAT TGTCTTTGCT AGGACCTCTC CTCAACAGAA GCTCATCATT 2340
   GTGGAGGGCT GCCAGCGGCA GGGTGCCATC GTGGCTGTCA CAGGGGATGG TGTCAATGAC 2400
   TCTCCAGCTT TGAAAAAGGC AGATATTGGG GTTGCCATGG GGATTGTTGG CTCGGATGTG 2460
   TCCAAGCAAG CTGCTGACAT GATTCTTCTG GATGACAACT TTGCCTCCAT CGTGACTGGA 2520
   GTAGAAGAAG GTCGTCTGAT ATTTGATAAC TTGAAGAAAT CCATTGCTTA CACCCTAACA 2580
   AGTAACATTC CGGAAATCAC CCCCTTCTTG ATATTTATTA TTGCAAACAT TCCACTGCCC 2640
   TGTGGCACCG TGACCATCCT CTGCATTGAC TTGGGCACTG ACATGGTTCC CGCCATCTCT 2700
   CTGGCCTATG AACAGGCTGA AAGTGACATC ATGAAGAGGC AGCCCAGAAA TCCCAAAACG 2760
   GACAAACTTG TGAACGAGCG TCTGATCAGC ATGGCCTATG GACAGATCGG TATGATCCAG 2820
   GCCCTGGGAG GCTTCTTCAC TTATTTTGTG ATTCTGGCTG AGAACGGTTT CCTGCCCTTT 2880
   CACCTGTTGG GCATCCGAGA GACCTGGGAT GACCGCTGGA TCAATGATGT GGAGGACAGC 2940
   TACGGGCAGC AGTGGACCTA CGAGCAGAGG AAGATTGTGG AGTTCACCTG CCACACGGCC 3000
   TTCTTTGTCA GTATCGTGGT AGTGCAGTGG GCTGACTTGG TCATCTGCAA GACCAGAAGG 3060
   AATTCTGTCT TCCAGCAGGG AATGAAGAAC AAGATCTTAA TATTTGGCCT CTTTGAAGAG 3120
   ACAGCTCTTG CTGCTTTCCT GTCCTACTGC CCTGGGATGG GTGCAGCCCT TAGGATGTAT 3180
   CCCCTCAAAC CTACTTGGTG GTTCTGTGCC TTCCCCTACT CCCTTCTCAT CTTCGTGTAT 3240
   GACGAGGTGC GGAAGCTCAT CATCAGGCGA CGCCCTGGCG GCTGGGTGGA GAAGGAAACC 3300
   TACTACTAGC CCACTGCCCT GCACGCCGTG GAACATTGTG CCACACACTG CACCTACCCC 3360
   TACCCCCCCT TTGTGTACTT CAAGTCTTGG AGCTCGGAAC TCTACCCTGG TAGGAAAGCA 3420
   CCAAAGCATG TGGGGATCCA GACGTCCTGG AATGAAGCAT GTAGCTGTAA TGGGGGGCGG 3480
   GGGGAGGGCT GCCCGAAAAA CACCGTGGAC GGGGACGACA GCGGGGAAGG TTTATATGTG 3540
   CCTTTTTGTT TTTGTAAAAA AGGAAAACCT GGAAAGACTG AAAGATTACG TTTTATATCT 3600
   GGATTTTTAC AAATAAAGAT GGCTATTATA ACGGAA 3636
(2) INFORMATION FOR SEQ ID NO: 7:
   (i) SEQUENCE CHARACTERISTICS:
      (A) LENGTH: 3636 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Rattus rattus
      (C) INDIVIDUAL ISOLATE: RAT
   (xi) SEQUENCE DESCRIPTION: SEQ ID NO: 7:
(2) INFORMATION FOR SEQ ID NO: 8:
   (i) SEQUENCE CHARACTERISTICS:
      (A) LENGTH: 3636 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: NKA alpha 1 subunit
      (C) INDIVIDUAL ISOLATE: rat
      (G) CELL TYPE: rat
   (xi) SEQUENCE DESCRIPTION: SEQ ID NO: 8:
   GGAGCCTCGG CGGGAGGAGG CGGACACGTG GCAGCGGCGG CGGCAGCGGC AGCAGCAGCG 60
   GCGGCAGCAG CGGCGGCCTC GGTCCGGGGC GCCGGCCGTC CTCCCTCTTT CCTCCGGCGG 120
   CAGCCCTAGT TCCCGCCTCT CGGCTCCCCC GGCTCCACTC TCCCAGCCGG GAGCTGCTCT 180
   CTCCTCTTTC TAGTCTCCAG CCACAGGACC CGGCGCGGGG CCCGCAGCGC CGCCACCATG 240
   GGGAAGGGGG TTGGACGAGA CAAGTATGAG CCCGCAGCTG TATCAGAACA TGGGGACAAG 300
   AAGAGCAAGA AGGCGAAGAA GGAAAGGGAC ATGGACGAAC TCAAGAAGGA AGTGTCTATG 360
   GACGACCATA AACTCAGCCT GGATGAACTC CATCGTAAAT ACGGAACAGA TTTGAGCCGA 420
   GGCCTAACAC CCGCAAGGGC CGCTGAGATC CTGGCTCGGG ATGGCCCCAA CGCCCTCACG 480
   CCCCCTCCCA CTACTCCCGA GTGGGTCAAA TTCTGTCGGC AGCTGTTCGG TGGCTTCTCC 540
   ATGTTACTGT GGATTGGAGC CATTCTTTGT TTCTTGGCTT ATGGCATCCG AAGTGCTACA 600
   GAAGAGGAAC CACCAAATGA TGATCTGTAC CTCGGGGTCG TGCTGTCTGC TGTCGTCATC 660
   ATAACTGGCT GTTTCTCCTA TTATCAAGAA GCAAAAAGCT CCAAGATCAT GGAATCCTTC 720
   AAGAACATGG TCCCTCAGCA AGCCCTCGTG ATTCGAAATG GAGAGAAGAT GAGCATCAAC 780
   GCAGAGGATG TCGTCGTTGG TGATCTGGTG GAGGTGAAGG GCGGAGACCG AATCCCTGCT 840
   GATCTCAGAA TCATATCTGC AAATGGCTGC AAGGTGGATA ACTCCTCACT CACTGGTGAA 900
   TCAGAACCCC AGACTCGGTC CCCGGATTTC ACAAACGAGA ACCCCTTGGA GACAAGGAAC 960
   ATTGCCTTCT TCTCAACCAA CTGTGTTGAA GGAACTGCAC GTGGCATCGT TGTGTACACT 1020
   GGGGATCGCA CCGTGATGGG CAGGATCGCC ACCCTTGCTT CTGGGCTGGA AGGCGGCCAG 1080
   ACCCCCATTG CTGAAGAAAT CGAGCACTTC ATCCACCTCA TCACGGGTGT GGCCGTGTTC 1140
   CTGGGGGTGT CTTTCTTCAT TCTCTCTCTG ATCCTTGAGT ACACCTGGCT CGAGGCTGTC 1200
   ATCTTCCTCA TTGGTATCAT CGTAGCCAAC GTGCCGGAAG GTTTGCTGGC CACCGTCACG 1260
   GTATGTCTGA CGCTCACTGC CAAGCGCATG GCGAGGAAGA ACTGCCTGGT GAAGAACCTG 1320
   GAAGCTGTGG AGACCTTGGG GTCCACATCC ACCATCTGCT CCGACAAGAC TGGAACTCTG 1380
   ACTCAGAACC GGATGACAGT GGCTCACATG TGGTTTGACA ATCAAATCCA TGAAGCTGAC 1440
   ACCACAGAGA ATCAGAGTGG GGTCTCCTTT GACAAGACGT CAGCCACCTG GTTCGCTCTG 1500
   TCCAGAATTG CTGGTCTCTG TAACAGGGCA GTGTTTCAGG CTAACCAAGA AAACCTGCCT 1560
   ATCCTTAAGC GTGCAGTAGC GGGAGATGCT TCCGAGTCGG CGCTCTTAAA GTGCATCGAG 1620
   GTCTGCTGTG GCTCCGTGAT GGAGATGAGG GAGAAGTACA CCAAGATAGT GGAGATTCCT 1680
   TTCAACTCCA CCAACAAGTA CCAGCTCTCC ATTCACAAGA ACCCAAACGC ATCGGAGCCT 1740
   AAGCACCTGC TAGTGATGAA GGGCGCCCCA GAAAGGATCC TGGACCGATG CAGTTCTATC 1800
   CTCCTCCACG GCAAGGAGCA GCCCCTGGAC GAAGAGCTGA AGGACGCCTT TCAGAATGCC 1860
   TACCTAGAGC TGGGGGGCCT TGGAGAGCGT GTGCTAGGTT TCTGCCACCT CCTTCTGCCT 1920
   GACGAACAGT TTCCCGAAGG CTTCCAGTTT GACACTGATG AAGTCAATTT CCCCGTGGAT 1980
   AACCTCTGCT TCGTGGGTCT TATCTCCATG ATTGACCCTC CTCGAGCTGC TGTCCCCGAT 2040
   GCTGTGGGCA AATGCCGCAG CGCTGGGATT AAGGTCATCA TGGTCACAGG AGACCATCCA 2100
   ATCACAGCCA AAGCCATTGC TAAGGGGGTG GGCATTATCT CAGAAGGTAA CGAGACCGTG 2160
   GAAGACATTG CTGCCCGCCT CAACATTCCA GTGAACCAGG TGAACCCCAG AGATGCCAAG 2220
   GCCTGTGTAG TACATGGCAG TGACTTGAAG GACATGACCT CTGAGGAGCT GGATGACATT 2280
   TTGCGGTACC ACACGGAGAT TGTCTTTGCT AGGACCTCTC CTCAACAGAA GCTCATCATT 2340
   GTGGAGGGCT GCCAGCGGCA GGGTGCCATC GTGGCTGTCA CAGGGGATGG TGTCAATGAC 2400
   TCTCCAGCTT TGAAAAAGGC AGATATTGGG GTTGCCATGG GGATTGTTGG CTCGGATGTG 2460
   TCCAAGCAAG CTGCTGACAT GATTCTTCTG GATGACAACT TTGCCTCCAT CGTGACTGGA 2520
   GTAGAAGAAG GTCGTCTGAT ATTTGATAAC TTGAAGAAAT CCATTGCTTA CACCCTAACA 2580
   AGTAACATTC CGGAAATCAC CCCCTTCTTG ATATTTATTA TTGCAAACAT TCCACTGCCC 2640
   CTGGGCACCG TGACCATCCT CTGCATTGAC TTGGGCACTG ACATGGTTCC CGCCATCTCT 2700
   CTGGCCTATG AACAGGCTGA AAGTGACATC ATGAAGAGGC AGCCCAGAAA TCCCAAAACG 2760
   GACAAACTTG TGAACGAGCG TCTGATCAGC ATGGCCTATG GACAGATCGG TATGATCCAG 2820
   GCCCTGGGAG GCTTCTTCAC TTATTTTGTG ATTCTGGCTG AGAACGGTTT CCTGCCCTTT 2880
   CACCTGTTGG GCATCCGAGA GACCTGGGAT GACCGCTGGA TCAATGATGT GGAGGACAGC 2940
   TACGGGCAGC AGTGGACCTA CGAGCAGAGG AAGATTGTGG AGTTCACCTG CCACACGGCC 3000
   TTCTTTGTCA GTATCGTGGT AGTGCAGTGG GCTGACTTGG TCATCTGCAA GACCAGAAGG 3060
   AATTCTGTCT TCCAGCAGGG AATGAAGAAC AAGATCTTAA TATTTGGCCT CTTTGAAGAG 3120
   ACAGCTCTTG CTGCTTTCCT GTCCTACTGC CCTGGGATGG GTGCAGCCCT TAGGATGTAT 3180
   CCCCTCAAAC CTACTTGGTG GTTCTGTGCC TTCCCCTACT CCCTTCTCAT CTTCGTGTAT 3240
   GACGAGGTGC GGAAGCTCAT CATCAGGCGA CGCCCTGGCG GCTGGGTGGA GAAGGAAACC 3300
   TACTACTAGC CCACTGCCCT GCACGCCGTG GAACATTGTG CCACACACTG CACCTACCCC 3360
   TACCCCCCCT TTGTGTACTT CAAGTCTTGG AGCTCGGAAC TCTACCCTGG TAGGAAAGCA 3420
   CCAAAGCATG TGGGGATCCA GACGTCCTGG AATGAAGCAT GTAGCTGTAA TGGGGGGCGG 3480
   GGGGAGGGCT GCCCGAAAAA CACCGTGGAC GGGGACGACA GCGGGGAAGG TTTATATGTG 3540
   CCTTTTTGTT TTTGTAAAAA AGGAAAACCT GGAAAGACTG AAAGATTACG TTTTATATCT 3600
   GGATTTTTAC AAATAAAGAT GGCTATTATA ACGGAA 3636

## Description

### Introduction

The present invention relates to a new method for selecting cells which involves a mutated naturally occuring life-essential enzyme, namely a (Na,K)-ATPase (EC 3.6.1.3). A mutated naturally occuring (Na,K)- ATPase or a subunit thereof, as well as their medical use is claimed.

In Herrera *et al.* (J. Cell. Biol. Vol. 105, pp. 1855-1865, 1987) three differentially expressed rat Na,K-ATPase a subunit isoforms was studied to investigate tissue-specific expression and primary structure. They found that the different isoforms were differentially expressed. For example, the α1 isoform was expressed in all fetal and adult tissue examined while the α2 isoform was predominantly expressed in brain and fetal heart. From the structural analysis Herrera *et al.* predicted a polytopic protein with seven membrane-spanning regions and two putative regions involved in ouabain binding. The interaction of Na,K-ATPase a subunit, primarily the al subunit, with ouabain is believed to be one of the explanations to resistance to ouabain in, for example, rats.

When transfecting genes to cells or individuals, a selection is often required and multiplication of the cells which have received the new gene is desirable. This can be done in many ways. Today a usual procedure is to simultaneously use genes which are resistant to antibiotics or cytostatics which makes the cells with the new gene resistant to these substances. It is then possible to make a selection between host cells which have received the antibiotic resistance gene and those host cells who have not by using the corresponding antibiotic. This is a well-known procedure for a person skilled in the art. (See e.g. Gene transfer and expression protocols. (Murray E.J, ed) Humana Press, New Jersey).

This procedure however have some disadvantages. It is expensive and may disturb the host cell due to the fact that an artificial gene has to be put in together with a certain selected gene. The cells must be selected using antibiotics or cytostatics. These antibiotic and cytostatic compounds might also interfere with other cellular mechanisms. It is not suitable to insert genes conferring antibiotic or cytostatic resistance to human cells in the case of gene therapy. A current problem with gene therapy today is that the efficiency is low; possibly due to the low level of gene transfected cells.

Accordingly, there is a need for a new method for selection of cells which lack these disadvantages.

### Summary of the invention

It has now been found, that by using a protein which is (Na,K)-ATPase or one of its α-subunits comprising the amino acid sequence : IFHANIPX₁PX₂GTVTILCID , wherein at least one of X₁ and X₂ is cysteine, as a marker for screening cells, most of the drawbacks listed above can be overcome. Preferably the protein comprises at least one of the following three amino acid sequences:
IFIIANIPCPLGTVTILCID, IFIIANIPLPCGTVTILCID or IFHANIPCPCGTVTILCID.

Accordingly a new selection system fulfilling the above mentioned needs has now been discovered. The system is based upon using a protein already existing in the cell protein as selection gene namely (Na,K)-ATPase; EC 3.6.1.3. This enzyme is essential for the cell and the gene is expressed in all mammalian cells. The (Na,K)-ATPase (a.k.a. Na+,K+-ATPase or NKA) is essential for the cell and it transports the sodium and Potassium ions over the cell membrane. Without the function of this enzyme the cell will die.

In this system, such a gene has been mutated in a particular manner which causes a loss in sensitivity to the substance ouabain (a cardiotonic steroid or glycoside). This substance is able to kill all cells which have not received the mutated protein and surviving cells are only cells which have received the mutated gene product. Ouabain normally depresses the activity of (Na,K)-ATPase.

There are advantages with this new system. No heterologous gene has to be added to the cells. This minor mutation is more natural than adding an artificial gene or making some major mutations. Moreover, the selection substance ouabain is also relatively cheap and stable. Ouabain is neither an antibiotic substance nor a cytostatic compound. In gene therapy, it will not be necessery to screen using antibiotic or cytostatic compounds. The possibilty is also given to purify and multiply only interesting cells for gene therapy (e.g. hematopoetic stemcells) with minimal intervention in the natural system of the cell. This new system gives an improved possibility of transporting new genes into cells from humans and animals. This can also improve the results in gene therapy and transplantation of e.g. hematopoetic stem cells, which have been subjected to gene therapy, to humans. Due to the fact that selection can be carried out with an enzyme, preferably NKA, which is already situated in the cell, an immunological answer on the transfected cells is minimized or eliminated when the cells are transplanted into a patient, e.g. when transplanting stem cells (not embryonic cells or germ cells) into a human. The risk for an immunological reaction to occur must be regarded to be much higher when using a selection gene which is normally not expressed in the cell. There is also a new possibility to study effects of cardiac glycosides (e.g. digitalis) on cells or whole animals who are resistant to these drugs. This ouabain-resistant enzyme could also be very useful when studying the enzyme or its biological effects.

We will now go into further details of the invention.

### Detailed description of the invention

The protein encoded by the gene as well as variants, subfragments and multiples of the protein having essentially the same antigenic and/or binding characteristics also constitutes an object of the present invention. The new protein is referred to as 799/801NKA. Preferably the amino acid sequence of the 799/801NKA should be at least 70% homologous, more prefarbly at least 85% homologous, still more preferably at least 90% homologous and most preferably at least 95% homologous to anyone of the amino acid sequences disclosed in SEQ ID NOS 1, 2, 3. Preferably 799/801NKA has either a cysteine in position 799 or in position 801 or a cysteine in both positions. When we call the protein life-essential later in this document we mean that the cell can not survive without its function

By "subfragment" is meant a part-fragment of the given protein having essentially the same antigenic and /or binding characteristics. By "variants" is meant proteins or peptides in which the original amino acid sequence has been modified or changed by insertion, addition, substitution, inversion, or exclusion of one or more amino acids By "multiples" is meant those proteins containing multiples of the whole original protein or those proteins containing multiples of subfragments and/or variants thereof.

The present invention also relates to nucleic acid sequences encoding 799/801 NKA. As utilized within the context of the present invention, nucleic acid sequences which encode 799/801 NKA are deemed to be substantially similar to those disclosed herein if: (a) the nucleic acid sequence is derived from the coding region of a native NKA gene (including, for example, variations of the sequences disclosed herein); (b) the nucleic acid sequence is capable of hybridization to nucleic acid sequences of the present invention under conditions of either moderate or high stringency (hybridization in 5 x SSPE containing 0.1% SDS and 0.1 mg/ml ssDNA, at 50-65°C depending on the probe length, or 10-20°C below the Tₘ of the probe; washing in 1 x SSPE, 0.1% SDS at 15-20°C below the Tₘ of the probe for moderate stringency, and in 0.1 x SSPE, 0.1% at 10°C below the Tₘ of the probe for high stringency conditions) (see Sambrook et al., Molecular Cloning: A Laboratory Manual, 2nd Ed., Cold Spring Harbor Laboratory Press, NY, 1989); or (c) nucleic acid sequences are degenerate as a result of the genetic code to the nucleic acid sequences defined in (a) or (b). Furthermore, although nucleic acid molecules are primarily referred to herein, as should be evident to one of skill in the art given the disclosure provided herein, a wide variety or related nucleic acid molecules may also be utilized in various embodiments described herein, including for example, RNA, nucleic acid analogues, as well as chimeric nucleic acid molecules which may be composed of more than one type of nucleic acid.

Within another aspect of the present invention, probes and primers are provided for detecting nucleic acids sequences which encode 799/801NKA. Within one embodiment of the invention, probes are provided which are capable of hybridizing to 799/801NKA nucleic acids (DNA or RNA). For purposes of the present invention, probes are "capable of hybridizing" to 799/801NKA nucleic acids if they hybridize to SEQ ID NO 5 to 8 under conditions of moderate or high stringency (see the section above concerning nucleic acid molecules, and Sambrook et al., supra); Preferably, the probe may be utilized to hybridize to suitable nucleotide sequences in the presence of 5 x SSPE, 0.1% SDS, and 0.1 mg/ml ssDNA at 10-20°C below the Tₘ of the probe. Subsequent washes may be performed in 1 x SSPE, 0.1% SDS at 15-20°C for conditions of moderate stringency, and in 0.1 x SSPE, 0.1% SDS al 10°C below the Tₘ of the probe for conditions of high stringency.

Probes of the present invention may be composed of either deoxyribonucleic acids (DNA) ribonucleic acids (RNA), nucleic acid analogues, or any combination of these, and may be as few as about 12 nucleotides in length, usually about 14 to 18 nucleotides in length, and possibly as large as the entire sequence which encodes 799/801NKA. Selection of probe size is somewhat dependent upon the use of the probe. For example, a long probe used under high stringency conditions is more specific, whereas a oligonucleotide carefully selected from the sequence can detect a structure of special interest.

Probes may be constructed and labeled using techniques which are well known in the art Shorter probes of, for example, 12 or 14 bases may be generated synthetically. Longer probes of about 75 bases to less than 1,5 kb are preferably generated by, for example, PCR amplification in the presence of labeled precursors such as ³²p-dCTP, digoxigenin-dUTP, or biotin-dATP. Probes of more than 1.5 kb are generally most easily amplified by transfecting a cell with a plasmid containing the relevant probe, growing the transfected cell into large quantities, and purifying the relevant sequence from the transfected cells (see San brook et al., supra).

Probes may be labeled by a variety of markers, including, for example, radioactive markers, fluorescent markers, enzymatic markers, and chromogenic markers. The use of 32p is particularly preferred for marking or labeling, a particular probe.

As noted above, nucleic acid probes of the present invention may be utilized to detect the presence of 799/801NKA nucleic acid molecules within a sample. However, if such nucleic acids molecules are present in only a limited number, then it may be beneficial to amplify the relevant sequence such that it may be more readily detected or obtained.

A variety of methods may be utilized in order to amplify a selected sequence, including, for example, RNA amplification (see Lizardi et al., Bio/Technology 6:1197-1202, 1988, Kramer et al., Nature 339:401-402, 1989; Lomeli et al., Clinical Chem. 35(91) 1826-1831, 1989; U.S. Patent No 4,786.600), and nucleic acid amplification utilizing Polymerase Chain Reaction ("PCR") (see U.S. Patent Nos. 4,683,195, 4,683,202, and 4,800,159) (see also U.S Patent Nos. 4,876,187, and 5,011,769, which describe an alternative detection/amplification system comprising the use of scissile linkages).

Within a particularly preferred embodiment, PCR amplilication is utilized to detect or obtain 799/801NKA nucleic acids. Briefly, as described in greater detail below, a nucleic acid sample is denatured at 95°C in order to generate single stranded nucleic acid. Specific primers, as discussed below, are then annealed at 37°C to 70°C, depending on the proportion of AT/GC in the primers. The primers are extended at 72°C with Taq polymerase in order to generate the opposite strand to the template. These steps constitute one cycle, which may be repeated in order to amplify the selected sequence. A nucleic acid sequence probe is necessary for the detection of the presence of the 799/801NKA, comprising at least XYZCCCXYZ, preferably TCCAXYZCCCXYZGGCACCG, derived from SEQ ID NO. 5-7 where XYZ is TGT or CTG and at least one of them is TGT and where this nucleic acid sequence probe has preferably a length of 9-100 nucleotides. A method for detecting the 799/801NKA in a cell, would comprise the steps of
a) rendering the DNA of the cell available for detection according to generally known methods;
b) carrying out a PCR detection using the probe above;
c) detecting the presence of amplification products formed in step b) by generally known methods.

A kit for detecting the presence of the 799/801NKA in a cell must comprise a nucleic acid sequence probe mentioned above.

Primers for the amplification of a cenain sequence should be selected from sequences which are highly specific and form stable duplexes with the target sequence The primers should also be non-complementary especially at the 3' end, should not form diners with themselves or other primers, and should not form secondary structures or duplexes with other regions of the nucleic acid. In general, primers of about 18 to 20 nucleotides are preferred, and may be easily synthesized using techniques well known in the art.

In another aspect, the present invention relates to vectors and host cells comprising the above mentioned nucleic acid sequences. The above described nucleic acid molecules which encode 799/801 NKA (or portions thereof) may be readily introduced into a wide variety of host cells. Representative examples of such host cells include plant cells, eukaryotic cells, and prokaryotic cells. Within preferred embodiments, the nucleic acid molecules or proteins are introduced into cells from a vertebrate or warm-blooded animal, such as a human, macaque, dog, cow, horse, pig, sheep, rat, hamster, mouse, or a fish, or any hybrid thereof (excluding human/animal hybrids).

The nucleic acid molecules (or vectors) may be introduced into host cells by a wide variety of mechanisms, including for example calcium phosphate-mediated transfection (Wigler et al., Cell 14:725, 1978), lipofection, gene gun (Corsaro and Pearson, Somatic Cell Gen. 1 603, 1981; Graham and Van der Eb, Virology 52:456, 1973), electroporation (Neumann et al., EMBO J. 1:841-845, 1982), retroviral, adenoviral, protoplast fusion-mediated transfection or DEAE-dextran mediated transfection (Ausubel et al., (eds.), Current Protocols in Molecular Biology John Wiley and Sons, Inc., NY, NY, 1987).

The nucleic acid molecules, antibodies, and proteins of the present invention may be labeled or conjugatad (either through covalent or non-covalent means) to a variety of labels or other molecules, including for example, fluorescent markers, enzyme markers, toxic molecules, molecules which are nontoxic but which become toxic upon exposure to a second compound, and radionuclides.

Representative examples of fluorescent labels suitable for use within the present invention include, for example, Fluorescein Isothiocyanate (FITC), Rodamine, Texas Red, Luciferase and Phycoerythrin (PE). Particularly preferred for use in flow cytometry is FITC which may be conjugated to purified antibody according to the method of Keltkamp in "Conjugation of Fluorescein Isothiocyanate to Antibodies. 1. Experiments on the Conditions of Conjugation," Immunology 18:865-873, 1970. (See also Keltkamp, "Conjugation of Fluoresscein Isothiocyanate to Antibodies. II. A Reproducible Method," Immunology 18:875-881, 1970; and Goding, "Conjugation of Antibodies with Fluorochromes: Modification to the Standard Methods," J. Immunol. Methods 13:215-226, 1970). For histochemical staining, HRP, which is preferred, may be conjugated to the purified antibody according to the method of Nakane and Kawaoi ("Peroxidase-Labeled Antibody: A New Method of Conjugation," J. Histochem. Cytochem. 22:1084-1091, 1974; see also, Tijssen and Kurstak, "Highly Efficient and Simple Methods for Preparation of Peroxidase and Active Peroxidase Antibody Conjugates for Enzyme Immunoassays," Anal. Biochem. 136;451-457, 1984).

Representative examples of enzyme markers or labels include alkaline phosphatase, horse radish peroxidase, and β-galactosidase. Representative examples of toxic molecules include ricin, abrin, diphtheria toxin, cholera toxin, gelonin, pokeweed antiviral proteein, tritin, Shigella toxin, and Pseudomonas exotoxin A. Representative examples of molecules which are nontoxic, but which become toxic upon exposure to a second compound include thymidine kinases such as HSVTK and VZVTK. Representative examples of radionuclides include Cu-64, Ga-67, CTa-68, Zr-89, Ru-97, Tc-99m, Rh-105, Pd-109, In-111, I-123, I-125, I-131, Re-186, Re-188, Au-198, Au-199, Pb-203, At-211, Pb-212 and Bi-212

As will be evident to one of skill in the art given the disclosure provided herein, the above described nucleic acid molecules, antibodies, proteins and peptides may also be labeled with other molecules such as colloidal gold, as well either member of a high affinity binding pair (e.g., avidin-biotin).

As noted above, the present invention also provides a variety of pharmaceutical compositions, comprising eucaryotic cells transformed with genes encoding 799/801/NKA, along with a pharmaceutically or physiologically acceptable carrier, excipients or diluents. Generally, such carriers should be nontoxic to recipients at the dosages and concentrations employed. Ordinarily, the preparation of such compositions entails combining the therapeutic agent with buffers, antioxidants such as ascorbic acid, low molecular weight (less than about 10 residues) polypeptides, proteins, amino acids, carbohydrates including glucose, sucrose or dextrins, chelating agents such as EDTA, glutathione and other stabilizers and excipients. Neutral buffered saline or saline mixed with nonspecific serum albumin are exemplary appropriate diluents. Such compositions may find use in gene therapy applications.

In addition, the pharmaceutical compositions of the present invention may be prepared for administration by a variety of different routes, including for example intraarticularly, intracranially, intradermally, intramuscularly, intraocularly, intraperitoneally, intrathecally, intravenously, subcutaneously or even directly into a tumor (for example, by stereotaxic injection). In addition, pharmaceutical compositions of the present invention may be placed within containers, along with packaging material which provides instructions regarding the use of such pharmaceutical compositions. Generally, such instructions will include a tangible expression describing the reagent concentration, as well as within certain embodiments, relative amounts of excipient ingredients or diluents (e,g., water, saline or PBS) which may be necessary to reconstitute the pharmaceutical composition.

As previosly mentioned, effective amounts of the protein or fragments or variants thereof can be used as active ingredients in pharmaceutical compositions possibly together with pharmaceutically acceptable excipients. Examples of suitable excipients are mannitol, laclose, starch, cellulose, glucose, etc., only to mention a few. The examples given of the adjuvant and the excipients are not to be regarded as limiting the invention

Accordingly, the invention is useful in, among all, the following fields:

*Genetical:* To select gene transfected cells for purifying and multiplying specific cell populations in the field of gene-therapy on humans and animals and also for screenig succesful in the area of transfections of cells in cell cultures. This gives the possibility, after the selection is made, to evaluate the efficiency of the transfection/gene therapy by method used. There is also a possibilty to store purified transfected cells for further use (e.g. at -70°C in liquid nitrogen). This procedure can be repeated to obtain a suitable amount or cells needed for therapy.

*Physiological:* The mutation/mutations in the protein, preferrably (Na,K)-ATPase, and its effects can be used in cell cultures and in non-human transgenetical animals in studies on a cellular, organ and fullbody level in connection with the use of effects of cardiac glycosides (e.g. digitalis).

in this system, a gene is used which is already expressed in all mammalian cells and which is essential for the survival of the cell. Earlier studies have shown that the activity of this enzyme can be influenced by the chemical substance ouabain (a cardiac glycoside) which is closely related to digitalis. The sensitivity for this influence depends on the structure of the enzyme and through mutations of the enzyme it can be altered (Lingrel J.B. Jour. Biol. Chem. 1994 pp. 10659-19662; Palais M., Jour Biol. Chem., 1996, pp. 14176-14182 Burns; E.L., Jour. Biol. Chem., 1996 pp15879-15883). In these studies the gene in lamb was used and it was shown that different mutations gave different results.

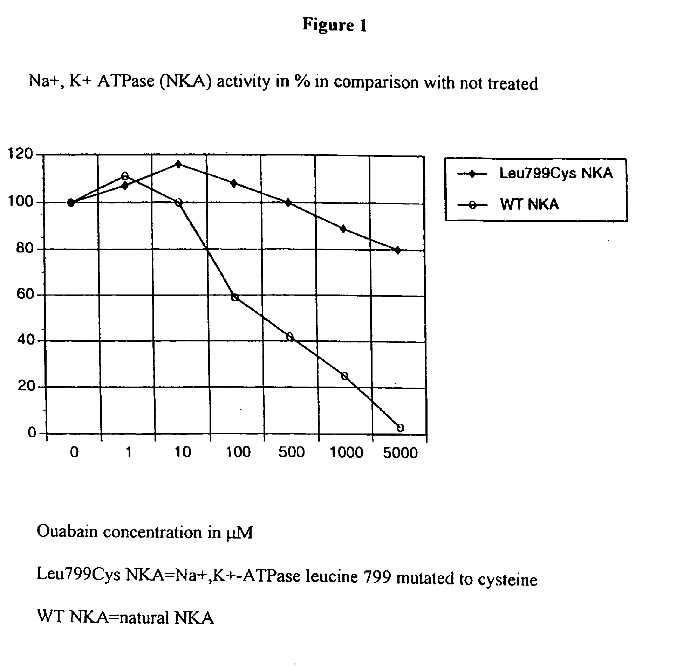

Here a specific mutation was made in the rat NKA alpha I subunit where the amino acid leucin-799 was altered to the amino acid cysteine in the same position in the gene coding for the alpha1-subunit of (Na,K)ATPase (NKA) in the rat (Figure 1) which unexpectedly resulted in an enzyme with an almost complete resistance to ouabain. Also experiments using the same NKA alpha 1 subunit but where the mutation had been done in position 801 instead of 799 from leucine to cysteine, result in the same effect as the one above. Cantley et al (Jour. Biol. Chem. 1994 pp 15358-61) have shown a mutation in murine and human NKA alpha 1-subunit and its importance for oubain resistance. They have reported about cells with an IC₅₀ for ouabain of approximately 5mM.

NKA is an integral membrane protein found in the cells of all higher eukaryotes and is responsible for translocating sodium and potassium ions across the cell membrane utilizing ATP as the driving force. The NKA is a member of the P-type class of ATPases which i n-cludes the sarcoplasmic reticulum and plasma membrane Ca2+-ATPases and the H+,K+-ATPases found in stomach and colon, in addition to several prokaryotic transport enzymes (Lingrel J., Jour. Biol. Chem., 1994, pp 19659-19662). These enzymes share a similar catalytic cycle that involves a phosphorylated protein intermediate.

The NKA is composed of two subunits in equimolar ratios. These are the alpha-subunit with a molecular mass of approximately 113 kDa and the smaller glycosylated beta-subunit with a protein portion accounting for 35 kDa of the over all molecular mass of 55 kDa. Isoforms exists for both the α ( α1, α2 and α3) and β (β1,β2 and β3) subunits. The α1 isoform occurs in most tissues, while the α2 isoform is predominant in skeletal muscle and is also detected in the brain and in the heart. The a 3 isoform is limited essentially to neural and cardiac tissue.

Due to the fact that all mammals (in particular humans, chicken, horses, pigs, sheep but also shrimps like Bufus marinus) have approximately the same sequence in this area i.e. 20 amino acids upstream and 20 amino acids downstream from this mutation it is very likely that a similar mutation in these species will render a similar enzyme with the same characteristics. It is also very likely that if a change of the leucin in position 801 to a cysteine occured together with a change in postion 799 from leucine to cysteine an enzyme would be obtained also with similar charcteristics as the NKA alpha 1 subunits with only one mutation in either position 799 or 801. However, this specific amino acid substitution is of great importance for the result. The unique and unexpected in this was that w c showed that the change of amino acid leucin 799 to cysteine in the rat gene coding for NKA alphal-subunit gives this drastic change in ouabain sensitvity. At ouabain concentrations up to 2mM, no significant inhibition of the enzyme could be detected. Also other cardiac glycosides could be thinkable such as: ouabagenin, digitoxin, digitogenin, digoxin, bufalin. The cardiac glycosides could possibly be used in concentrations from 1nM to 5000 µM, preferably 1µM to 5000µM, to separate cells comprising the mutated NKA from cells not comprising the mutated NKA where the cells are exposed to a cardiac glycoside and the surviving cells are recovered.

The very high similarity in amino acid sequence between species and Isoforms of this enzyme, in the region of interest, strengthen the possibility that these mutations can be used in several other species and isoforms different from rat Na+,K+-ATPase alpha1 subunit. This gives the invention wider fields of availabillity. It is also very likely that by using the mutated NKA subunit from each species a selection of cells can be done in that particular species by using the NKA subunit in that particular species. This will probably result in the most natural selection system of today.

An example of the amino acid (a a) similarity in this domain (62 aa upstream and 82 aa downstream of the mutations). The similarity (homology) is given as % of rat alpha 1 subunit sequence:

| | |
|---|---|
| Human alpha1 | 100% |
| Human alpha3 | 97,2% |
| Horse alpha1 | 99,3% |
| Pig alpha1 | 99,3% |
| Pig alpha3 | 98,6% |
| Chicken alpha1 | 98,6% |
| Chicken alpha2 | 99,3% |
| Chicken alpha3 | 96,5% |
| Sheep alpha1 | 98,6% |
| Rat alpha2 | 98,6% |
| Rat alpha3 | 96,5% |

These different subunits in a suitable mix would be useful when preparing non-human transgenetic animals with different characteristics.

Earlier the natural gene coding for the NKA alphal-subunit had been used (Ouabr-vector, see Research Cataloge 1994-1995 from PharMingen page 271) for transfection selection, but this has had limitations due to that its resistance had only been partial and only a few celltypes could be transfected. The NKA alpha 1 subunit here came from rat which only makes it useful when dealing with rats.This new enzyme (NKA-Leu799Cys, here we call it 799NKA) with a total unsensitivity for ouabain makes this mutated protein and its corresponding gene interesting in several areas both in research and clinically.

The fact that the a 1 subunit of NKA is expressed in all types of cells can be used by using the natural genomic areas/regions which controls the transcription activity of the gene. One example of a genetic structure which can be a part of the construction which is intended to be used in the area of gene therapy is the following:

Where A,D,E and F are the natural regulatory genomic structures uppstream and downstream of the genes, alternatively fundamentally activated regulatory structures,

B is a"selection gene" which is a naturally occuring gene for the cell which is expressed in all cells and has one for the cell essential function (in our example I subunit of NKA). This gene is mutated so that it has got one or more characteristics altered (not dangerous for the cell) which makes the cell possible to distinguish from the naturally occuring gene and untransfected cells (in our example (NKA L799C/L801C). Where z represents the mutation in the "selection gene".
and C is the gene which the most important for gene therapy itself.

In US 4474893 there has been suggested to use hybridomas resistant to ouabain for selection Ouabain-resistant cells would be able to survive levels of ouabain which kill normal ouabain-sensitive cells. Ouabain-resistance may be used as a selection marker by itself, or in combination with other markers. It is also very likely that by mutating other essential proteins in cells you could acquire a similar possibility to make selection among cells i.e. cells which have received this minor, natural, mutation from ordinary cells. Other essential proteins in the cells are e.g. metabolic enzymes and DNA or RNA polymerases.

An explanation to why this mutation to cysteine in position 799 causes this huge difference when using the NKA in high concentrations of ouabain might be that there is a cys=cys disulfide bridge formed (Kirley, T.L. et , Jour. Biol. Chem 1986, pp 4525-4528). The suggested binding between a cysteine and ouabain could be abolished in view of the invention described in this application due to the formation of this cyc=cys bridge. This theory shall not however limit the invention in any form.

The invention will now be described in more detail with reference to the accompanying drawing, in which

Figure 1 shows (Na,K)ATPase activity in percent in comparison with not treated vs ouabain concentration in µM. The Leu799Cys NKA is a (Na,K)-ATPase where the leucine 799 is mutated to cysteine. WT NKA is natural (Na,K)-ATPase.

SEQ ID NO:1 relates to a (Na,K)ATPase (NKA) alpha 1 subunit with its leucine 879 (corresponds to 799) mutated to cysteine. SEQ ID NO:2 relates to a (Na,K)ATPase (NKA) alpha 1 subunit with its leucine 881 (corresponds to 801) mutated to cysteine. SEQ ID NO:3 relates to a (Na,K)ATPase (NKA) alpha 1 subunit with its leucine 879 (corresponds to 799) mutated to cysteine and also its leucine 881 (corresponds to 801) mutated to cysteine. SEQ ID NO.4 is natural NKA alpha 1 subunit. SEQ ID NO. 5-8 are the corresponding DNA-sequences to the above mentioned amino acid sequences. NO: corresponds to NO:5, NO:2 corresponds to NO:6,NO:3 corresponds to NO:7 and NO:4 corresponds to NO:8. SEQ ID NO:s 1-4 include an 80 amino acid start peptide and SEQ ID NO:s 5-8 include a 240 nucleic acid long start codon.

The invention will now be further described with reference to the following examples.
These examples are only given for the purpose of illustration and are not intended to limit the scope of the invention claimed herein.

### Experimental Procedures

### MUTAGENESIS

To introduce the mutation leucine 799 to cysteine (L799C) or leucine 801 to cysteine (L801C) in the rat Na+,K+-ATPase al subunit, a cDNA sequence of the gene inserted into a PBS+ BlueScript vector was used. And the mutations was introduced using a polymerase chain reaction mutation strategy (Pfu DNA polymerase from Stratagene, La Jolla, California). To produce the fragments needed to create each mutation, PCR was carried out with the following primer pairs: to create the L799C mutation:
5'-ATGATTGACCCTCCTCGAGCTGCT-'3 5'-AATAAATATCAAGAAGGGGGTGAT-'3;
5'-GGCCTGGATCATACCGATCTGT-'3 5'-ATTGCAAACATTCCATGTCCCCTGG-'3; to create the L801C mutation:
5'-ATGATTGACCCTCCTCGAGCTGCT-'3 5'-GTTTGCAATAATAAATATCAAGAAGGG-'3 5'-GGCCTGGATCATACCGATCTGT-'3
5'-ATTCCACTGCCCTGTGGCACCGTGA-'3 The following PCR reaction cycles were used: 96°C for 3 min followed by 30 cycles of 57°C for 30 sec, 72°C for 1 min and 95°C for 20 sec, and finally 72°C for 5 min. The fragments were subjected to a polynucleotide
kinase reaction (Boehringer Mannheim, Germany) for 30 min at 37°C and then ligated with the ligase chain reaction (LCR) (Pfu DNA ligase, Stratagene, La Jolla, California) with wild type rat Na+,K+-ATPase al cDNA as a template. The following LCR reaction cycles were used: 96°C for 2 min and 60°C for 2 min followed by 30 cycles of 96°C for 20 sec and 60°C for 20 sec.

The mutated fragments was purified by agarose gel electrophoresis, digested with Bcl 1 and Eco 47 (New England Biolabs Inc. USA) restriction enzymes and substituted for the wild type fragment. Each complete clone was sequenced with an Applied Biosystems Taq Dye Deoxy Terminator Cycle Sequencing Kit in a Perkin-Elmer Cetus DNA Thermal Cycler 9600 to confirm the mutation and exclude other mutations.

### Transfection:

Wild type and the mutant Na+,K+-ATPase a1 were subcloned in a CMV- or adenovirus promotor containing mammalian expression vector and stably transfected into COS7 (monkey), LLC-PK1 (pig) and MDCK (dog) cells using the Calcium Phosphate method (described by Okayama, H., and Chen, C. (1991) in Gene transfer and expression protocols (Murray EJ, ed) pp 15-21, Humana Press, New Jersey). Cells were grown in Dulbecco's modified Eagle's medium (DMEM) supplemented with 10% fetal-calf serum and 5% penicillin/ streptomycin in 37°C humified air with 5.3 % CO₂. The cells, which are derived from different spices than rat, express a relative high ouabain-sensitive Na+,K+-ATPase. Selection of transfected clones was achieved as described (Fisone, G., Cheng, S., Nairn, A., Czernik, A., Hemmings, H., Hvvg, J., Bertorello, A., Kaiser, R., Bergman, T., Jvrnvall, H., Aperia, A. and Greengard, P. (1994)J. Biol. Chem. 269, 9368-9373, Vilsen, B. FEBS Letters (1992) 314(3) 301-307 and Vilsen, B. Biochemistry (1993) 32(48): 13340-13349). Briefly, cells were grown in 10 or up to 500 µM ouabain for 3-4 weeks, and the medium was changed every third day. Untransfected cells died within 2-3 days in ouabain medium, while cells expressing the relatively ouabain-insensitive wild type or mutant rat Na+,K+-ATPase a1 subunit survived. Following this ouabain selection procedure, several hundred single clones with approximately the same growth rate were pooled and replated in DMEM containing 10 or 500 µM ouabain. We selected clones of cells, expressing wild type and mutant Na+,K+-ATPase, that had similar levels of transfected Na+,K+ -ATPase activity. The abundance of Na+,k+-ATPase, as determined by immunoblotting, was also within the same range in both cell types. In piloi studies we could clearly distinguish between ouabain-sensitive (endogenous) and ouabain-insensitive (transfected) Na+,K+-ATPase populations that were completely inhibited (the wild type enzyme) by ouabain concentrations of 10⁻⁵ M and 5x10⁻³ M, respectively- Approximately 60% of Na+,K+-ATPase was resistent to 10⁻⁵ M ouabain and was considered to be rat wild type or mutated Na+,K+-ATPase. Furthermore, the expression of wild type and mutant Na+,K+-ATPase mRNA was in each protocol verified by RT-PCR with RNase free DNase (Pharmacia Biotech, Sweden) treated total RNA. ATP hydrolysis:

Cells expressing wild type and mutant Na+,K+-ATPase were seeded (3.0 x 10⁵ cells/well) in 30 mm diameter wells and grown to confluence. Cells were lysed by treatment with 1mM Tris-HCl pH 7.5 on ice for 15 min and a crude membrane fraction was prepared as described (Horiuchi, A., Takeyasu, K., Mouradian, M., Jose, P. and Felder, R. (1993) Mol. Pharmacol. 43, 281-285). The membrane fraction was frozen in aliquots and stored overnight at -70°C. Na+,K+-A TPase-dependent ATP hydrolysis was measured in triplicate as described (Fisone, G., Cheng, S., Nairn, A., Czemik, A., Hemmings, H., Hvvg, J., Bertorello, A., Kaiser, R., Bergman, T., Jvrnvall, H., Aperia, A. and Greengard, P. (1994) J. Biol. Chem. 269, 9368-9373). Transfected-Na+,K+-ATPase activity was determined as the difference between ATPase activity at 10⁻⁵ M and at stepwise increasing concentrations of ouabain.

These experimental procedures gave these results in three different examples:

### Example 1

After the mutation (leucin 799 to cysteine) in the (Na,K)-ATPase had been created it was transfected to a mammalian cell line (COS-7). The activity of the normal and the mutated enzyme was studied (see Figure 1). You can see that the mutated enzyme keeps its activity at almost 100%, while the activity of the natural enzyme is inhibited 85-90% at a concentration of 2mM ouabain.

### Example 2

A cell surviving study was done where the cells transfected with the mutated enzyme survived a longtime treatment with 500µM ouabain while the cells transfected with the natural enzyme died at a concentration of 500µM.

### Example 3

LLC-PK1 (cells from pig kidney) and MDCK (cells from dog kidney) have also been transfected and survival studies have shown that these cell types also survive, multiply and keep their natural exterior at ouabain concentrations up to 500 µM.

It is generally accepted that the LLC-PK1 cells have a tendency to differentiate when the cell population is grown to confluence and the cells form circular configurations with transporting properties similar to epithelial cells. Transfection of the mutated (L799C and/or L801C) rat Na+,K+-ATPase alpha1 subunit into the LLC-PK1 cells did not seem to alter these cells growing and morphological characteristics, in as high as 500x10⁻⁶ M ouabain.
Transfection of the COS 7 and MDCK cell lines did not affect these cells growing and morphology as in the case for the LLC-PK1 cells.

Untransfected cells died within a few days in 1-50x10⁻⁶ M ouabain, wild type transfected cells died within a few days in a 500x10⁻⁶ M ouabain while mutant transfected cells survived as high as at least 1x10⁻³ M ouabain.

### Exemple 4

### Cell Attachment

Cell attachment on fibronectin coted substrates was assayed in microtiterwells (Costar). All wells were preincubated over night at 4°C with 5 microg/ml concentration of fibronectin. Before adding cells,each well was preincubated with 1% BSA for 30 min in 37°C and later washed with serum free DMEM. Untransfected COS-7 cells and COS-7 cells expressing wild type or L799C Na+,K+- ATPase were grown in a 3 microCi/ml H3-thymidine containing medium supplemented with 10% fetal calf serum and harvested after approximately 72 hours at 60-80% confluence. Approximately 25 000 cells /wells were seeded in serum free DMEM. Cells were first incubated 15 min with different concentrations of oubain and then allowed to attach to fibronectin (5 µg/ml) coated substrates during 30 min of incubation in 37°C and later washed with serum free DMEM. Radioactivity was measured in a Wallac Scintillation counter. Results are presented as % remaining radioactivity compared to untreated.

### Results cell attachment

This system is based upon differences in cell attachment between transfected and untransfected cells when they are exposed to oubain. Since we here can show that an inhibition of Na+,K+-ATPase activity lead to an concomitant inhibition of cell attachment this can be used to select transfected cells, ready to be used in different applications, within a few hours instead of several weeks. This can speed up the procedure several fold. During selection with different concentrations of oubain (i.e. inhibition of Na+, K+-ATPase), cell attachment was decreased dose dependent in untransfected cells but no change in attachment was noted in transfected cells. When complete inhibition of Na+,K+ATPase was achieved almost no attachment was seen. The effect on attachment was seen within minutes. This procedure could be repeated to obtain a 100% selection efficiency within hours.

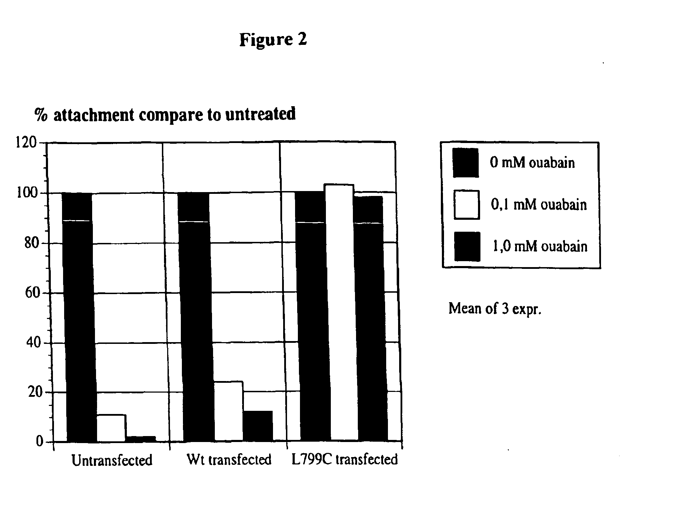

The results in figure 2 are presented as means of 3 experiments. One can clearly see a "loss of attachment" in untransfected and wild type transfected cells compared to L799C Na+,K+-ATPase transfected cells.

## Claims

1. A protein which is (Na,K)-ATPase or one of its alpha subunits comprising the amino acid sequence:
IFIIANIPX₁PX₂GTVTILCID
wherein at least one of X₁ and X₂ is cysteine.

2. The protein according to claim 1 which comprises at least one of the following three amino acid sequences selected from the group consisting of: IFIIANIPCPLGTVTILCID, IFIIANIPLPCGTVTILCID or IFIIANIPCPCGTVTILCID.

3. The protein according to anyone of claims 1-2 having an amino acid sequence according to anyone of SEQ ID NO: 1, SEQ ID NO: 2 or SEQ ID NO: 3.

4. A nucleic acid sequence encoding a protein according to anyone of claims 1-3.

5. A vector comprising the nucleic acid of claim 4.

6. A cell comprising a vector according to claim 5.

7. A nucleic acid molecule comprising a nucleic sequence according to claim 4 for medical use.

8. A vector for use in gene therapy comprising a nucleic acid molecule according to claim 4 together with another physiologically interesting nucleic acid sequence.

9. A method for detecting whether a certain transfection or transformation of an eukaryotic cell with a gene of interest is successful, comprising:
a) transfecting or transforming the cell with a vector comprising a nucleic acid according to claim 4 as well as the gene of interest;
b) incubating the cells from step a) with a suitable amount, preferably 1 - 5000 µM, of a cardiac glycoside such as ouabain, ouabagenin, digitoxin, digitogenin, digoxin and bufalin;
c) contacting the cells from step b) with a fibronectin-coated solid matrix;
d) detecting any cells that has bound to the matrix.

10. The method according to claim 9, wherein the incubation in step b) lasts for 5 - 30 minutes, and preferably for 15 minutes, and where step c) is carried out during 5 - 60 minutes, and preferably during 30 minutes.

11. The method according to anyone of claims 9 and 10, wherein the cardiac glycoside is ouabain.

12. A method for screening animal cells comprising a protein according to anyone of claims 1 - 3, comprising the steps of
a) exposing the cells to a cardiac glycoside such as ouabain, ouabagenin, digitoxin, digitogenin, digoxin and bufalin;
b) recovering surviving cells.

## Patentansprüche

1. Ein Protein, das eine (Na,K)-ATPase oder eine ihrer alpha-Untereinheiten ist, das die Aminosäuresequenz
IFIIANIPX₁PX₂GTVTILCID,
umfasst, wobei wenigstens ein X₁ und X₂ Cystein ist.

2. Das Protein gemäß Anspruch 1, das wenigstens eine der drei folgenden Aminosäuresequenzen umfasst, die aus der Gruppe ausgewählt werden, die besteht aus: IFIIANIPCPLGTVTILCID, IFIIANIPLPCGTVTILCID oder IFIIANIPCPCGTVTILCID.

3. Das Protein gemäß einem der Ansprüche 1-2 mit einer Aminosäuresequenz gemäß SEQ ID NO: 1, SEQ ID NO: 2 oder SEQ ID NO: 3.

4. Eine Nukleinsäuresequenz, die für ein Protein gemäß einem der Ansprüche 1-3 kodiert.

5. Ein Vektor, der die Nukleinsäure gemäß Anspruch 4 umfasst.

6. Eine Zelle, die einen Vektor gemäß Anspruch 5 umfasst.

7. Ein Nukleinsäuremolekül, das zur medizinischen Verwendung eine Nukleinsäuresequenz gemäß Anspruch 4 umfasst.

8. Ein Vektor für die Verwendung in der Gentherapie, der ein Nukleinsäuremolekül gemäß Anspruch 4 zusammen mit einer anderen physiologisch interessanten Nukleinsäuresequenz umfasst.

9. Ein Verfahren zum Detektieren, ob eine bestimmte Transfektion oder Transformation einer eukaryotischen Zelle mit einem entsprechenden Gen erfolgreich ist, das umfasst:
a) Transfizieren oder Transformieren der Zelle mit einem Vektor, der sowohl eine Nukleinsäure gemäß Anspruch 4 als auch das entsprechende Gen umfasst;
b) Inkubieren der Zellen aus Schritt a) mit einer geeigneten Menge, bevorzugt 1-5000 µM eines Herzglykosids, wie zum Beispiel Ouabain, Ouabagenin, Digitoxin, Digitogenin, Digoxin und Bufalin;
c) In Kontakt bringen der Zellen aus Schritt b) mit einer mit Fibronektin beschichteten festen Matrix;
d) Detektieren irgendeiner Zelle, die an die Matrix gebunden ist.

10. Das Verfahren gemäß Anspruch 9, wobei die Inkubation in Schritt b) 5-30 Minuten und bevorzugt 15 Minuten andauert und wobei Schritt c) für 5-60 Minuten und bevorzugt für 30 Minuten durchgeführt wird.

11. Das Verfahren gemäß einem der Ansprüche 9 und 10, wobei das Herzglykosid Ouabain ist.

12. Ein Verfahren zum Untersuchen tierischer Zellen, das ein Protein gemäß einem der Ansprüche 1-3 umfasst, das die Schritte umfasst
a) Aussetzen der Zellen gegenüber einem Herzglykosid, wie zum Beispiel Ouabain, Ouabagenin, Digitoxin, Digitogenin, Digoxin und Bufalin;
b) Wiedergewinnen der überlebenden Zellen.

## Revendications

1. Protéine qui est la (Na,K)-ATPase ou une de ses sous-unités alpha, comprenant la séquence d'acides aminés :
IFIIANIPX₁PX₂GTVTILCID
dans laquelle au moins un de X₁ et X₂ est la cystéine.

2. Protéine selon la revendication 1, qui comprend au moins une des trois séquences d'acides aminés suivantes choisies dans le groupe constitué par : IFIIANIPCPLGTVTILCID, IFIIANIPLPCGTVTILCID, ou IFIIANIPCPCGTVTILCID.

3. Protéine selon l'une quelconque des revendications 1 et 2, ayant une séquence d'acides aminés selon l'une quelconque de SEQ ID NO : 1, SEQ ID NO : 2 ou SEQ ID NO : 3.

4. Séquence d'acide nucléique codant pour une protéine selon l'une quelconque des revendications 1 à 3.

5. Vecteur comprenant l'acide nucléique selon la revendication 4.

6. Cellule comprenant un vecteur selon la revendication 5.

7. Molécule d'acide nucléique comprenant une séquence d'acide nucléique selon la revendication 4, pour une utilisation médicale.

8. Vecteur destiné à être utilisé en thérapie génique, comprenant une molécule d'acide nucléique selon la revendication 4, conjointement avec une autre séquence d'acide nucléique physiologiquement intéressante.

9. Procédé de détection de la réussite ou non d'une certaine transfection ou transformation d'une cellule eucaryote avec un gène d'intérêt, qui comprend :
a) la transfection ou la transformation de la cellule avec un vecteur comprenant un acide nucléique selon la revendication 4 ainsi que le gène d'intérêt ;
b) l'incubation des cellules de l'étape a) avec une quantité adaptée, de préférence de 1 à 5 000 µM, d'un glycoside cardiaque tel que l'ouabaïne, l'ouabagénine, la digitoxine, la digitogénine, la digoxine et la bufaline ;
c) la mise en contact des cellules de l'étape b) avec une matrice solide enrobée de fibronectine ;
d) la détection de toute cellule qui s'est liée à la matrice.

10. Procédé selon la revendication 9, dans lequel l'incubation dans l'étape b) dure 5 à 30 minutes, et de préférence 15 minutes, et où l'étape c) est réalisée pendant 5 à 60 minutes, et de préférence pendant 30 minutes.

11. Procédé selon l'une quelconque des revendications 9 et 10, dans lequel le glycoside cardiaque est l'ouabaïne.

12. Procédé de criblage de cellules animales, comprenant une protéine selon l'une quelconque des revendications 1 à 3, comprenant les étapes consistant à :
a) exposer les cellules à un glycoside cardiaque tel que l'ouabaïne, l'ouabagénine, la digitoxine, la digitogénine, la digoxine et la bufaline ;
b) récupérer les cellules survivantes.
